# EUROPEAN PATENT SPECIFICATION

(11) **EP 4 714 491 B1**
(45) Date of publication and mention of the grant of the patent: **14.10.2026**
(21) Application number: 25201986.4
(22) Date of filing: 12.09.2025
(51) Int. Cl.: A61N 1/37, A61N 1/375, A61B 5/35, A61N 1/372, A61B 5/352

(54) **DEVICES FOR IMPROVED EVOKED RESPONSE DETECTION AND PACEMAKER CAPTURE MANAGEMENT**
VORRICHTUNGEN ZUR VERBESSERTEN ERKENNUNG VON EVOZIERTEN REAKTIONEN UND ZUR VERWALTUNG DER HERZSCHRITTMACHERERFASSUNG
DISPOSITIFS POUR UNE DÉTECTION AMÉLIORÉE DE RÉPONSE ÉVOQUÉE ET POUR UNE GESTION DE CAPTURE DE STIMULATEUR CARDIAQUE

(30) Priority: 24.09.2024 US 202463698347 P; 11.09.2025 US 202519326284
(43) Date of publication of application: 25.03.2026
(73) Proprietor: Pacesetter, Inc., Sylmar, CA 91342 (US)
(72) Inventor: Bornzin, Alexander R, Sylmar, CA 91342 (US); Badie, Nima, Sylmar, CA 91342 (US); Yang, Weiqun, Sylmar, CA 91342 (US)
(74) Representative: Barker Brettell Sweden AB

(56) References cited:
- EP-A2- 4 324 514
- US-A1- 2015 306 401
- US-A1- 2022 080 210
- US-A1- 2022 401 744

## Description

### TECHNICAL FIELD

The present technology relates to implantable medical devices (IMDs), and more particularly, IMDs that have pacing capabilities, such as pacemakers, and exemplary methods of the use of such IMDs.

### BACKGROUND

In pacemakers that are configured to pace an atrial cardiac chamber, detection of an atrial evoked response (AER), in response to delivery of an atrial pacing pulse, is of great value. The presence of an atrial evoked response is indicative of an atrial pacing pulse causing atrial capture. In a pacemaker that is capable of performing "atrial autocapture" the pacemaker may automatically and gradually reduce an atrial stimulus amplitude to determine the stimulus amplitude at which atrial capture is lost, which provides an estimate for an atrial capture threshold. Once a pacemaker determines the atrial capture threshold, the pacemaker can automatically set the atrial stimulus amplitude to be equal to the atrial capture threshold plus a small safety margin. Providing atrial autocapture is very valuable because the atrial stimuli safety margin may be minimized, which reduces battery current drain and thereby prolongs pacemaker longevity. This is especially critical for leadless pacemakers, because the battery takes up most of the volume of leadless pacemakers, and small size is important for leadless pacemakers, especially those that are implanted transvenously and left to reside inside the heart.

Atrial autocapture depends on atrial evoked response detection, but atrial evoked response detection is not a trivial endeavor. Atrial evoked response detection is challenging because of the small amplitude of a typical atrial evoked response. Indeed, a typical atrial evoked response is within the range of only about 1.0 millivolts (mV) to about 3.5 mV in amplitude, and typically has a duration of about 50 milliseconds or less.

When a pacing pulse is delivered, current is passed through a cathode electrode (e.g., a tip electrode of a leadless pacemaker) to an anode electrode (e.g., a ring or battery case electrode of the leadless pacemaker), and an electrode-to-electrolyte interface behaves like a capacitor and charges up to tens or hundreds of millivolts. After the pacing pulse is turned off and an active discharge is turned off, there is residual charge left on the electrode-to-electrolyte interface that creates a decaying potential between the electrodes. In a leadless pacemaker, the electrode-to-electrolyte interfaces at both the battery case electrode and the tip electrode charge up, leaving behind a decaying polarization potential that superimposes on the atrial evoked response. This polarization potential is often sufficiently large that the atrial evoked response is dwarfed and thereby totally obscured by the polarization potential.

Furthermore, an atrial evoked response typically includes a very early initial negative deflection that occurs within only a few milliseconds of the cathodic pacing pulse. Capturing the initial leading-edge may help to achieve the maximum deflection of the entire evoked response, thus enhancing evoked response detection. However, the active discharge of the output capacitor (also known as the pacing capacitor) that is used to generate the pacing pulse typically takes place for about 7 to 15 milliseconds following the end of the pacing pulse. The active discharge process therefore superimposes on the early negative atrial evoked response, which obscures the atrial evoked response. Making the active discharge short enough (e.g., 3 to 4 milliseconds following the end of the atrial pacing pulse) to detect the full atrial evoked response is possible, but has negative consequences. On the helpful side, the active discharge cancels electrode polarization. However, when the active discharge is too short, 3 to 4 milliseconds of polarization is not adequately cancelled, resulting in the atrial evoked response being concealed by the electrode polarization. Additionally, excess atrial electrode polarization can lead to lack of ventricular pacing support caused by crosstalk in a ventricular sensing channel, potentially inhibiting ventricular pacing. Crosstalk mitigation may necessitate ventricular safety pacing, also known as committed ventricular pacing, which wastes battery current and thereby shortens the longevity of the ventricular pacemaker.

Various techniques have been developed to attempt to mitigate electrode polarization, and thereby improve atrial evoked response detection. For example, coating the electrodes with titanium nitride (TiN), iridium oxide (IrOx), or platinum black, or combinations thereof, dramatically reduces electrode polarization. Indeed, pacemaker designers have used these coatings on pacemaker electrodes to reduce electrode polarization and thereby improve evoked response detection and even improve stimulation thresholds and efficiency.

Further, various techniques have been developed to improve the detection of an atrial evoked response in the presence of electrode polarization. For example, rather than simply comparing amplitudes of a portion of a sensed cardiac signal to an amplitude threshold, the portion of the sensed cardiac signal can be digitized and summed to extract an atrial evoked response integral that is compared to an integral threshold. If the atrial evoked response integral exceeds the integral threshold, then the pacemaker detects an atrial evoked response. However, such an integral technique does not work where an atrial evoked response is of sufficiently small amplitude that it remains obscured by electrode polarization after-potentials. Even more complex techniques have also been developed. For example, in one technique, about twenty digitized samples of a portion of a sensed cardiac signal are obtained and then correlated with a stored template for an atrial evoked response. Pearson's correlation coefficient may be used, but is very mathematically intensive requiring many multiplications. Alternatively, Kendell's tau correlation can be used to test the relationship between the template and the evoked response, wherein Kendell's tau correlation does not multiply, but rather, sorts and compares sample amplitudes. Unfortunately, frequently executed correlations (whether it is Pearson's or even Kendell's tau) is a significant burden on processor duty cycle and battery current drain. Preferably, a simpler process, such as differentiation of the atrial evoked response by performing twenty or fewer subtractions would be a better approach. The use of such a differentiation technique that relies on subtractions have been proven to be very effective for detecting a ventricular evoked response, each is typically about 3 to 4 time larger in amplitude than an atrial evoke response. However, because an atrial evoked response often has a very small amplitude, the use of such a differentiation technique that relies on subtractions is not always effective for detecting an atrial evoked response.

Various leadless pacemakers directed at capture detection are known from US 2015/306401 A1, EP 4 324 514 A2, US 2022/401744 A1 and US 2022/080210 A1.

As can be appreciated from the above discussion, it would be beneficial if further techniques were available for reducing adverse effects of electrode polarizations, and more generally, improving detections of evoked responses, especially atrial evoked responses, when performing autocapture and/or other types of capture detection.

### SUMMARY OF THE PRESENT INVENTION

The present invention concerns a leadless pacemaker as defined in claim 1. Preferred features and embodiments are defined by the dependent claims.

Methods described hereinafter are not claimed as such and only of exemplary nature. They are presented for better understanding the invention which is defined by the appended claims.

### SUMMARY OF THE PRESENT DISCLOSURE

Embodiments described hereinafter are of exemplary nature and presented for better understanding the invention which is defined by the appended claims.

Certain embodiments of the present technology related to an LP configured to be implanted in or on a cardiac chamber of a heart, the LP comprising: multiple electrodes; memory; a pulse generator configured to deliver, using at least two electrodes of the multiple electrodes, electrical stimulation pulses to the cardiac chamber; a sensing circuit configured to sense, using at least two electrodes of the multiple electrodes, an EGM indicative of electrical activity of the heart; and a controller communicatively coupled to the memory, the pulse generator, and the sensing circuit. In accordance with certain embodiments, the controller is configured to: cause delivery to the cardiac chamber, using the pulse generator and at least two electrodes of the multiple electrodes, of one or more capturing electrical stimulation pulses having a test pulse energy level that is above a capture threshold; produce a capture plus polarization artifacts template based on one or more portions of the sensed EGM within one or more respective windows following when the one or more capturing electrical stimulation pulses was/were delivered; save, within a portion of the memory, the capture plus polarization artifacts template corresponding to the test pulse energy level; cause delivery to the cardiac chamber, using the pulse generator and at least two electrodes of the multiple electrodes, of one or more pacing electrical stimulation pulses having a pacing pulse energy level that is less than the test pulse energy level; determine a difference signal between an evoked response morphology of the sensed EGM and the capture plus polarization artifacts template, wherein the evoked response morphology of the sensed EGM corresponds to one or more portions of the sensed EGM within one or more respective windows following when the one or more pacing electrical stimulation pulses having the pacing pulse energy level was/were delivered; and determine based on the difference signal whether loss of capture occurred responsive to the one or more pacing electrical stimulation pulses having the pacing energy level that is less than the test pulse energy level.

In accordance with certain embodiments, the controller, in response to determining that loss of capture of the cardiac chamber failed to occur responsive to the one or more pacing electrical stimulation pulses having the pacing pulse energy level that is less than the test pulse energy level, is configured to test a decreased pacing pulse energy level by: causing delivery to the cardiac chamber, using the pulse generator and at least two electrodes of the multiple electrodes, of one or more pacing electrical stimulation pulses having the decreased pacing pulse energy level; determining a further difference signal between a further evoked response morphology of the sensed EGM and the capture plus polarization artifacts template, wherein the further evoked response morphology of the sensed EGM corresponds to one or more portions of the sensed EGM within one or more respective windows following when the one or more pacing electrical stimulation pulses having the decreased pacing pulse energy level was/were delivered; and determining based on the further difference signal whether loss of capture of the cardiac chamber occurred responsive to the one or more pacing electrical stimulation pulses having the decreased pacing energy level.

In accordance with certain embodiments, the controller is configured to: in response to determining that loss of capture of the cardiac chamber failed to occur (i.e., in responding to determining that capture occurred) responsive to the one or more pacing electrical stimulation pulses having the decreased pacing energy level, test one or more further decreased pacing pulse energy levels until loss of capture the cardiac chamber is detected; in response to detecting loss of capture, identify as an updated capture threshold a last decreased pacing energy level that caused capture; and cause delivery to the cardiac chamber, using the pulse generator and at least two of the multiple electrodes, of further pacing electrical stimulation pulses having an updated pacing pulse energy level that exceeds by a safety margin the updated capture threshold.

In accordance with certain embodiments, the controller is configured to save in one or more further portions of the memory the updated capture threshold or the updated pacing pulse energy level, or both.

In accordance with certain embodiments, the controller is configured to: in response to determining that loss of capture of the cardiac chamber failed to occur responsive to the one or more pacing electrical stimulation pulses having the decreased pacing energy level, test one or more further decreased pacing pulse energy levels until loss of capture the cardiac chamber is detected; and in response to detecting loss of capture, cause delivery to the cardiac chamber, using the pulse generator and at least two of the multiple electrodes, of further pacing electrical stimulation pulses having an updated pacing pulse energy level that exceeds by a safety margin a last decreased pacing energy level that caused capture.

In accordance with certain embodiments, the controller is configured to cause delivery of the one or more capturing electrical stimulation pulses outside of one or more refractory periods corresponding to one or more windows following one or more depolarizations of the cardiac chamber.

In accordance with certain embodiments, the controller is configured to determine, based on the difference signal, whether loss of capture of the cardiac chamber occurred, by: determining an area under the curve of the difference signal or of a first derivative of the difference signal; comparing the area under the curve to a corresponding threshold; and determining that loss of capture of the cardiac chamber occurred when the area under the curve exceeds the corresponding threshold.

In accordance with certain embodiments, the controller is configured to determine the area under the curve by adding up positive values of the difference signal or the first derivative of the difference signal.

In accordance with certain embodiments, the controller is configured to determine based on the difference signal whether loss of capture of the cardiac chamber occurred, by: determining a first derivative of the difference signal; identifying a maximum peak of the first derivative of the difference signal; identifying a window of the first derivative of the difference signal that is centered about the maximum peak; determining an area under the curve of the window of the first derivative of the difference signal that is centered about the maximum peak; comparing the area under the curve to a corresponding threshold; and determining that loss of capture of the cardiac chamber occurred when the area under the curve exceeds the corresponding threshold.

In accordance with certain embodiments, the controller is configured to determine, based on the difference signal, whether loss of capture of the cardiac chamber occurred, by: determining a metric of the difference signal or of a first derivative the difference signal, wherein a larger the metric a greater the difference between the evoked response morphology of sensed EGM and the polarization artifacts template, and a smaller the metric a lesser the difference between the evoked response morphology of sensed EGM and the polarization artifacts template; comparing the metric to a corresponding threshold; and determining that loss of capture of the cardiac chamber occurred when the metric exceeds the corresponding threshold.

In accordance with certain embodiments, the LP comprises an atrial LP configured to be implanted in or on an atrial chamber.

The present technology also relates to an exemplary method for use by an LP implanted in or on a cardiac chamber of a heart, wherein the LP includes multiple electrodes and memory, and wherein the LP uses at least two of the multiple electrodes for delivering electrical stimulation pulses to the cardiac chamber and at least two of the multiple electrodes for sensing an EGM indicative of electrical activity of the heart, the method comprising: (a) delivering to the cardiac chamber, using at least two of the multiple electrodes, one or more capturing electrical stimulation pulses having a test pulse energy level that is above a capture threshold; (b) producing a capture plus polarization artifacts template based on one or more portions of the sensed EGM within one or more respective windows following when the one or more capturing electrical stimulation pulses was/were delivered; (c) saving, within a portion of the memory, the capture plus polarization artifacts template corresponding to the test pulse energy level; (d) delivering to the cardiac chamber, using at least two of the multiple electrodes, one or more pacing electrical stimulation pulses having a pacing pulse energy level that is less than the test pulse energy level; (e) determining a difference signal between an evoked response morphology of the sensed EGM and the capture plus polarization artifacts template, wherein the evoked response morphology of the sensed EGM corresponds to one or more portions of the sensed EGM within one or more respective windows following when the one or more pacing electrical stimulation pulses having the pacing pulse energy level was/were delivered; and (f) determining based on the difference signal whether loss of capture of the cardiac chamber occurred responsive to the one or more pacing electrical stimulation pulses having the pacing energy level that is less than the test pulse energy level.

In accordance with certain examples, the method further comprises: (g) in response to determining that loss of capture of the cardiac chamber failed to occur responsive to the one or more pacing electrical stimulation pulses having the pacing pulse energy level, testing a decreased pacing pulse energy level by repeating each of at least steps (d) through (f), such that the decreased pacing pulse energy level is used at the repeated step (d), and such that the determining at step (f) is whether loss of capture of the cardiac chamber occurred responsive to one or more pacing electrical stimulation pulses having the decreased pacing pulse energy level.

In accordance with certain examples, the method further comprises: in response to determining that loss of capture of the cardiac chamber failed to occur responsive to the one or more pacing electrical stimulation pulses having the decreased pacing energy level, testing one or more further decreased pacing pulse energy levels until loss of capture the cardiac chamber is detected; in response to detecting loss of capture, identifying as an updated capture threshold a last decreased pacing energy level that caused capture; and delivering to the cardiac chamber, using two of the multiple electrodes, further pacing electrical stimulation pulses having an updated pacing pulse energy level that exceeds by a safety margin the updated capture threshold.

In accordance with certain examples, the method further comprises: saving in one or more further portions of the memory the updated capture threshold or the updated pacing pulse energy level, or both.

In accordance with certain examples, the method further comprises: in response to determining that loss of capture of the cardiac chamber failed to occur responsive to the one or more pacing electrical stimulation pulses having the decreased pacing energy level, testing one or more further decreased pacing pulse energy levels until loss of capture the cardiac chamber is detected; and in response to detecting loss of capture, delivering to the cardiac chamber further pacing electrical stimulation pulses having an updated pacing pulse energy level that exceeds by a safety margin a last decreased pacing energy level that caused capture.

In accordance with certain examples, the method is periodically performed by the LP in order to update the capture threshold for the LP implanted in or on the cardiac chamber, and wherein the method further comprises: after the capture threshold is updated, updating the pacing pulse energy level based on the capture threshold such that the pacing pulse energy level exceeds the capture threshold by a specified safety margin; and delivering to the cardiac chamber, using the electrodes, further pacing electrical stimulation pulses having the pacing pulse energy level that has been updated.

In accordance with certain examples, at step (a) the delivering comprises delivering the one or more capturing electrical stimulation pulses having the test pulse energy level outside of one or more refractory periods corresponding to one or more windows following one or more depolarizations of the cardiac chamber.

In accordance with certain examples, at step (f) the determining comprises: determining an area under the curve of the difference signal or of a first derivative of the difference signal; comparing the area under the curve to a corresponding threshold; and determining that loss of capture of the cardiac chamber occurred when the area under the curve of the difference signal exceeds the corresponding threshold.

In accordance with certain examples, the determining the area under the curve includes adding up positive values of the difference signal or the first derivative of the difference signal.

In accordance with certain examples, at step (f) the determining comprises: determining a first derivative of the difference signal; identifying a maximum peak of the first derivative of the difference signal; identifying a window of the first derivative of the difference signal that is centered about the maximum peak; determining an area under the curve of the window of the first derivative of the difference signal that is centered about the maximum peak; comparing the area under the curve to a corresponding threshold; and determining that loss of capture of the cardiac chamber occurred when the area under the curve exceeds the corresponding threshold.

In accordance with certain examples, at step (f) the determining comprises: determining a metric of the difference signal or of a first derivative the difference signal, wherein a larger the metric a greater the difference between the evoked response morphology of sensed EGM and the polarization artifacts template, and a smaller the metric a lesser the difference between the evoked response morphology of sensed EGM and the polarization artifacts template; comparing the metric to a corresponding threshold; and determining that loss of capture of the cardiac chamber occurred when the metric exceeds the corresponding threshold.

In accordance with certain examples, the method further comprises: the LP that performs the method comprises an atrial LP implanted in or on an atrial chamber.

This summary is not intended to be a complete description of the embodiments of the present technology. Other features and advantages of the embodiments of the present technology will appear from the following description in which some exemplary embodiments have been set forth in detail, in conjunction with the accompanying drawings and claims.

### BRIEF DESCRIPTION OF DRAWINGS

Embodiments of the present technology relating to both structure and method of operation may best be understood by referring to the following description and accompanying drawings, in which similar reference characters denote similar elements throughout the several views:
FIG. 1 illustrates a system formed in accordance with certain embodiments herein as implanted in a heart.
FIG. 2 is a block diagram of a single LP in accordance with certain embodiments herein.
FIG. 3 illustrates an LP in accordance with certain embodiments herein.
FIG. 4 is a timing diagram demonstrating one embodiment of i2i communication for a paced event.
FIG. 5 is a timing diagram demonstrating one embodiment of i2i communication for a sensed event.
FIG. 6 is a high level flow diagram used to summarize methods of the present technology for providing capture management in manners that compensate for polarization artifacts and provide for efficient use of limited memory available in IMDs.
FIGS. 7A, 7B, and 7C are flow diagrams used to describe additional details of one of the steps introduced in FIG. 6, according to various embodiments of the present technology.
FIG. 8 includes graphs of EGMs used to illustrate how embodiments of the present technology, summarized with reference to the high level flow diagram of FIG. 6, provide for capture management in manners that compensate for polarization artifacts.
FIG. 9 is a high level flow diagram used to summarize further methods of the present technology for providing capture management.
FIGS. 10A, 10B, and 10C are flow diagrams used to describe additional details of one of the steps introduced in FIG. 9, according to various embodiments of the present technology.
FIG. 11 shows a block diagram of one embodiment of an IMD (e.g., LP or an ICD) that is implanted into the patient as part of the implantable cardiac system in accordance with certain embodiments herein.

### DETAILED DESCRIPTION

Certain embodiments of the present technology relate to implantable medical devices (IMDs), and exemplary methods of their use, that provide for capture management in manners that compensate for polarization artifacts and provide for efficient use of limited memory that is typically available in IMDs.

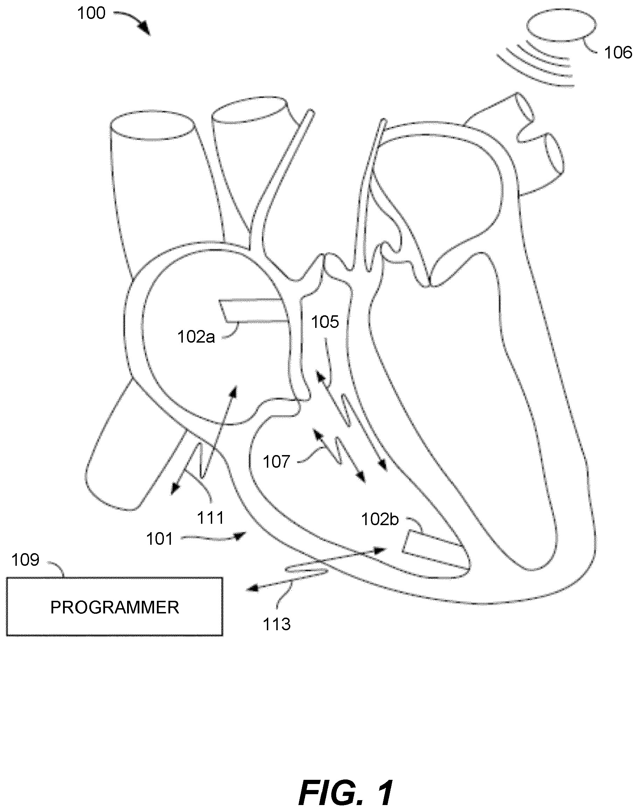

Before providing additional details of the specific embodiments of the present technology mentioned above, an example system in which embodiments of the present technology can be used will first be described with reference to FIGS. 1-5. More specifically, FIGS. 1-5 will be used to describe an example cardiac pacing system, wherein pacing and sensing operations can be performed by multiple medical devices, which may include one or more leadless cardiac pacemakers, a non-vascular implantable cardioverter defibrillator (ICD), such as a subcutaneous-ICD (S-ICD), and/or a programmer to reliably and safely coordinate pacing and/or sensing operations. A leadless cardiac pacemaker can also be referred to more succinctly herein as a leadless pacemaker (LP). Where a cardiac pacing system includes a non-vascular ICD (e.g., an S-ICD), the non-vascular ICD may perform certain sensing operations and may communicate with one or more LPs by sending and/or receiving messages to and/or from one or more LPs, as can be appreciated from the below discussion. Where a cardiac pacing system includes a programmer, the programmer may be used to program one or more IMDs, download information to one or more IMDs, and/or upload information from one or more IMDs, as can be appreciated from the below description.

FIG. 1 illustrates a system 100 that is configured to be implanted in a heart 101. The system 100 includes two or more leadless pacemakers (LPs) 102a and 102b located in different chambers of the heart 101. LP 102a is located in a right atrium, while LP 102b is located in a right ventricle. LPs 102a and 102b communicate with one another to inform one another of various local physiologic activities, such as local intrinsic events, local paced events and the like. LPs 102a and 102b may be constructed in a similar manner, but operate differently based upon which chamber the LP 102a or 102b is located. The LPs 102a and 102b may sometimes be referred to collectively herein as the LPs 102, or individually as an LP 102.

In certain embodiments, LPs 102a and 102b communicate with one another, and/or with an ICD 106, by conductive communication through the same electrodes that are used for sensing and/or delivery of pacing therapy. The LPs 102a and 102b may also be able to use conductive communication to communicate with an external device, e.g., a programmer 109, having electrodes placed on the skin of a patient within with the LPs 102a and 102b are implanted. The LPs 102a and 102b can each also include an antenna that would enable them to communicate with one another, the ICD 106 and/or an external device using RF communication. While only two LPs 102a and 102b are shown in the system 100 of FIG. 1, it is possible that more than two LPs can be implanted in a patient. For example, to provide for bi-ventricular pacing and/or cardiac resynchronization therapy (CRT), in addition to having LPs implanted in the right atrial (RA) chamber and the right ventricular (RV) chamber, a further LP can be implanted in the left ventricular (LV) chamber.

In some embodiments, one or more LP 102 can be co-implanted with the ICD 106 and/or an insertable cardiac monitor (ICM), not shown in FIG. 1. Each LP 102 uses two or more electrodes located within, on, or within a few centimeters of the housing of the pacemaker, for pacing and sensing at the cardiac chamber, for bidirectional conductive communication with one another, with the programmer 109, and the ICD 106.

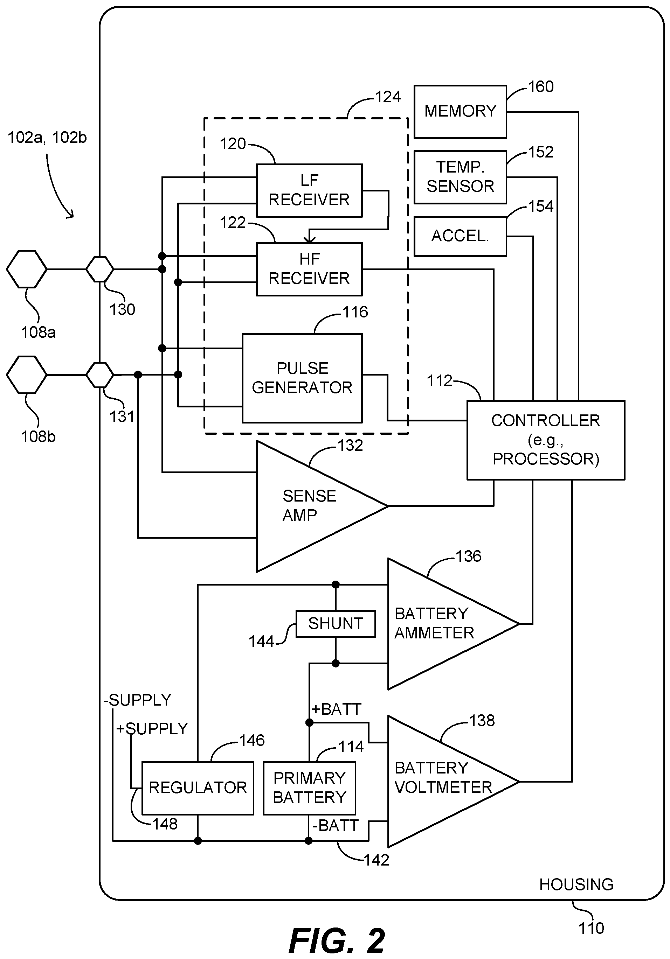

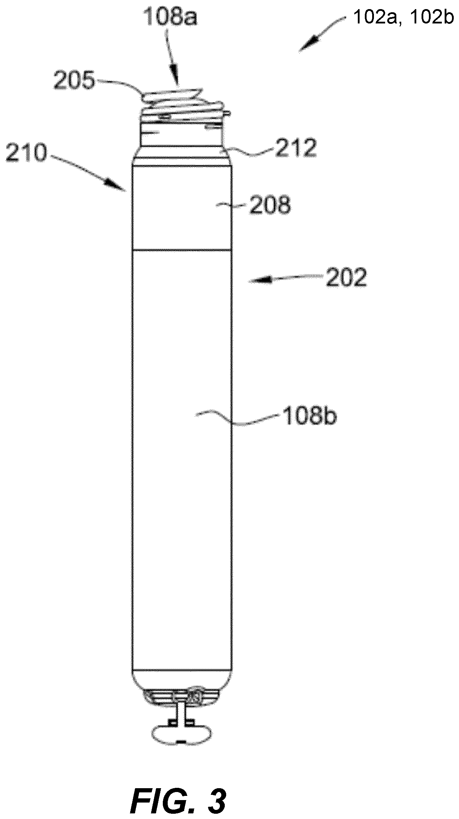

Referring to FIG. 2, a block diagram shows an embodiment for portions of the electronics within LPs 102a, 102b. One or more of LPs 102a and 102b include at least two leadless electrodes configured for delivering cardiac pacing pulses, sensing evoked and/or natural cardiac electrical signals, and preferably uni-directional or bi-directional conductive communication. In FIG. 2 (and FIG. 3) the two electrodes shown therein are labeled 108a and 108b. Such electrodes can be referred to collectively as the electrodes 108, or individually as an electrode 108. An LP 102, or other type of IMD, can include more than two electrodes 108, depending upon implementation. In certain embodiments, such as but not limited to where an LP 102 includes only two electrodes 108, the LP 102 uses the same electrodes 108 for delivering electrical stimulation pulses to the cardiac chamber and for sensing an EGM indicative of electrical activity of the heart. In embodiments where an LP 102 includes more than two electrodes (i.e., three or more electrodes), the LP may use different electrodes for delivering electrical stimulation pulses to the cardiac chamber than for sensing an EGM indicative of electrical activity of the heart. In still other embodiments, where an LP 102 includes more than two electrodes, the LP 102 may use a first set of the electrodes (e.g., first and second electrodes) for delivering electrical stimulation pulses to the cardiac chamber and use a second set of the electrodes (e.g., first and third electrodes) that differs from the first set for sensing an EGM indicative of electrical activity of the heart. An LP 102 can include three or more electrodes, e.g., by including more than one distal or tip electrode, more than one ring electrode, and/or at least one proximal electrode (e.g., on the proximal end opposite the distal most electrode 108a).

In FIG. 2, each of the LPs 102a, 102b is shown as including first and second receivers 120 and 122 that collectively define separate first and second conductive communication channels 105 and 107 (FIG. 1), (among other things) between LPs 102a and 102b. Although first and second receivers 120 and 122 are depicted, in other embodiments, each LP 102a, 102b may only include the first receiver 120, or more generally may include only a single receiver that is configured to receive conductive communication signals. It is also possible that an LP 102 may include additional receivers other than first and second receivers 120 and 122. As will be described in additional detail below, the pulse generator 116 can function as a transmitter that transmits i2i communication signals using the electrodes 108. In certain embodiments, LPs 102a and 102b may communicate over more than just first and second conductive communication channels 105 and 107. In certain embodiments, LPs 102a and 102b may communicate over one common communication channel 105. More specifically, LPs 102a and 102b can communicate conductively over a common physical channel via the same electrodes 108 that are also used to deliver pacing pulses and/or the same electrodes 108 that are also used to sense electrical activity of the heart.

The receivers 120 and 122 can also be referred to, respectively, as a low frequency (LF) receiver 120 and a high frequency (HF) receiver 122, because the receiver 120 is configured to monitor for one or more signals within a relatively low frequency range (e.g., below 250 KHz) and the receiver 122 is configured to monitor for one or more signals within a relatively high frequency range (e.g., above 250 KHz). In certain embodiments, the receiver 120 (and more specifically, at least a portion thereof) is always enabled and monitoring for a wakeup notice, which can simply be a wakeup pulse, within a specific low frequency range (e.g., between 1 KHz and 250 KHz); and the receiver 122 is selectively enabled by the receiver 120. The receiver 120 is configured to consume less power than the receiver 122 when both the first and second receivers are enabled. Accordingly, the receiver 120 can also be referred to as a low power receiver 120, and the receiver 122 can also be referred to as a high power receiver 122. The low power receiver 120 is incapable of receiving signals within the relatively high frequency range (e.g., above 250 KHz), but consumes significantly less power than the high power receiver 122. This way the low power receiver 120 is capable of always monitoring for a wakeup notice without significantly depleting the battery (e.g., 114) of the LP 102. In accordance with certain embodiments, the high power receiver 122 is selectively enabled by the low power receiver 120, in response to the low power receiver 120 receiving a wakeup notice, so that the high power receiver 122 can receive the higher frequency signals, and thereby handle higher data throughput needed for effective i2i communication without unnecessarily and rapidly depleting the battery 114 of the LP 102 (which the high power receiver 122 may do if it were always enabled).

Since the receivers 120, 122 are used to receive conductive communication messages, the receivers 120, 122 can also be referred to as conductive communication receivers. In certain embodiments, each of the LPs 102 includes only a single conductive communication receiver.

In accordance with certain embodiments, when one of the LPs 102a and 102b senses an intrinsic event or delivers a paced event, the corresponding LP 102a, 102b preferably transmits an implant event message to the other LP 102a, 102b. For example, when an atrial LP 102a senses/paces an atrial event, the atrial LP 102a transmits an implant event message including an event marker indicative of a nature of the event (e.g., intrinsic/sensed atrial event, paced atrial event). When a ventricular LP 102b senses/paces a ventricular event, the ventricular LP 102b transmits an implant event message including an event marker indicative of a nature of the event (e.g., intrinsic/sensed ventricular event, paced ventricular event). In certain embodiments, each LP 102a, 102b transmits an implant event message to the other LP 102a, 102b preceding the actual pace pulse so that the remote LP can blank its sense inputs in anticipation of that remote pace pulse (to prevent inappropriate crosstalk sensing). The above describe implant event messages are examples of i2i messages.

The implant event messages may be formatted in various manners. As one example, each event message may include a leading trigger pulse (also referred to as an LP wakeup notice, wakeup pulse or wakeup signal) followed by an event marker. The notice trigger pulse (also referred to as the wakeup notice, wakeup pulse or wakeup signal) is transmitted over a first channel (e.g., with a pulse duration of approximately 10 µs to approximately 1ms and/or within a fundamental frequency range of approximately 1 KHz to approximately 100 KHz). The notice trigger pulse indicates that an event marker is about to be transmitted over a second channel (e.g., within a higher frequency range). The event marker can then be transmitted over the second channel.

The event markers may include data indicative of one or more events (e.g., a sensed intrinsic atrial activation for an atrial located LP, a sensed intrinsic ventricular activation for a ventricular located LP). The event markers may include different markers for intrinsic and paced events. The event markers may also indicate start or end times for timers (e.g., an AV interval, a blanking interval, etc.). Optionally, the implant event message may include a message segment that includes additional/secondary information.

Optionally, the LP (or other IMD) that receives any i2i communication message from another LP (or other IMD) or from an external device may transmit a receive acknowledgement indicating that the receiving LP (or other IMD) received the i2i communication message. In certain embodiments, where an IMD expects to receive an i2i communication message within a window, and fails to receive the i2i communication message within the window, the IMD may transmit a failure-to-receive acknowledgement indicating that the receiving IMD failed to receive the i2i communication message. In other words, an LP can receive a message from another LP that includes an indicator (e.g., an error code) in its payload, or header, that indicates to the LP that the other LP failed to receive an expected message from the LP. Other variations are also possible and within the scope of the embodiments described herein.

The event messages enable the LPs 102a, 102b to deliver synchronized therapy and additional supportive features (e.g., measurements, etc.). To maintain synchronous therapy, each of the LPs 102a and 102b is made aware (through the event messages) when an event occurs in the chamber containing the other LP 102a, 102b. Some embodiments described herein provide efficient and reliable processes to maintain synchronization between LPs 102a and 102b without maintaining continuous communication between LPs 102a and 102b. In accordance with certain embodiments herein, low power event messages/signaling may be maintained between LPs 102a and 102b synchronously or asynchronously.

For synchronous event signaling, LPs 102a and 102b may maintain synchronization and regularly communicate at a specific interval. Synchronous event signaling allows the transmitter and receivers in each LP 102a,102b to use limited (or minimal) power as each LP 102a, 102b is only powered for a small fraction of the time in connection with transmission and reception. For example, LP 102a, 102b may transmit/receive (Tx/Rx) communication messages in time slots having duration of 10-20µs, where the Tx/Rx time slots occur periodically (e.g., every 10-20ms).

LPs 102a and 102b may lose synchronization, even in a synchronous event signaling scheme. As explained herein, features may be included in LPs 102a and 102b to maintain device synchronization, and when synchronization is lost, LPs 102a and 102b undergo operations to recover synchronization. Also, synchronous event messages/signaling may introduce a delay between transmissions which causes a reaction lag at the receiving LP 102a, 102b. Accordingly, features may be implemented to account for the reaction lag.

During asynchronous event signaling, LPs 102a and 102b do not maintain communication synchronization. During asynchronous event signaling, one or more of receivers 120 and 122 of LPs 102a and 102b may be "always on" (always awake) to search for incoming transmissions. However, maintaining LP receivers 120, 122 in an "always on" (always awake) state presents challenges as the received signal level often is low due to high channel attenuation caused by the patient's anatomy. Further, maintaining the receivers awake will deplete the battery 114 more quickly than may be desirable.

The asynchronous event signaling methods avoid risks associated with losing synchronization between devices. However, the asynchronous event signaling methods utilize additional receiver current between transmissions. For purposes of illustration only, a non-limiting example is described hereafter. For example, the channel attenuation may be estimated to have a gain of 1/500 to 1/10000. A gain factor may be 1/1000th. Transmit current is a design factor in addition to receiver current. As an example, the system may allocate one-half of the implant communication current budget to the transmitter (e.g., 0.5µA for each transmitter). When LP 102a, 102b maintains a transmitter in a continuous on-state and the electrode load is 500 ohms, a transmitted voltage may be 2.5V. When an event signal is transmitted at 2.5V, the event signal is attenuated as it propagates and would appear at LP 102a, 102b receiver as an amplitude of approximately 0.25mV.

To overcome the foregoing receive power limit, a pulsed transmission scheme may be utilized in which communication transmissions occur correlated with an event. By way of example, the pulsed transmission scheme may be simplified such that each transmission constitutes a single pulse of a select amplitude and width.

In accordance with certain embodiments herein, LPs 102a and 102b may utilize multi-stage receivers that implement a staged receiver wakeup scheme in order to improve reliability yet remain power efficient. Each of LPs 102a and 102b may include first and second receivers 120 and 122 that operate with different first and second activation protocols and different first and second receive channels. For example, first receiver 120 may be assigned a first activation protocol that is "always on" (also referred to as always awake) and that listens over a first receive channel that has a lower fundamental frequency range/pulse duration (e.g., 1 KHz to 100 KHz / 10 µs to approximately 1 ms) as compared to the fundamental frequency range (e.g., greater than 100 KHz / less than 10 µs per pulse) assigned to the second receive channel.

The first receiver 120 may optionally maintain the first channel active (awake) at all times (including when the second channel is inactive (asleep)) in order to listen for messages from a remote LP. The second receiver 122 may optionally be assigned a second activation protocol that is a triggered protocol, in which the second receiver 122 becomes active (awake) in response to detection of trigger events over the first receive channel (e.g., when the incoming signal corresponds to the LP wakeup notice, activating the second channel at the local LP). The terms active, turned on, awake and enabled are used interchangeably herein.

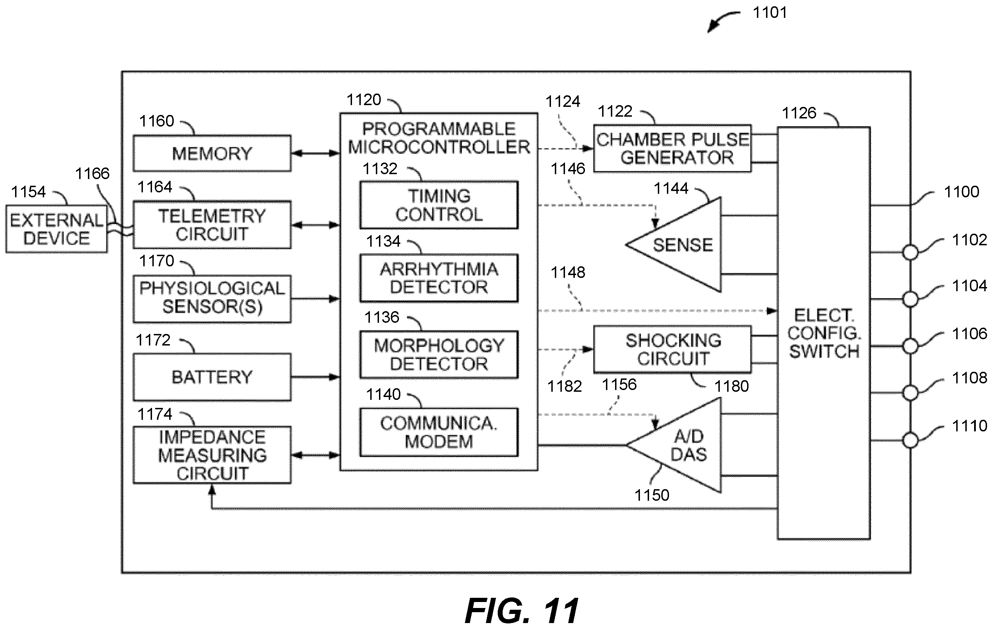

Still referring to FIG. 2, each LP 102a, 102b is shown as including a controller 112 and a pulse generator 116. The controller 112 can include, e.g., a microprocessor (or equivalent control circuitry), RAM and/or ROM memory, logic and timing circuitry, state machine circuitry, and I/O circuitry, but is not limited thereto. The controller 112 can further include, e.g., timing control circuitry to control the timing of the stimulation pulses (e.g., pacing rate, atrio-ventricular (AV) delay, atrial interconduction (A-A) delay, or ventricular interconduction (V-V) delay, etc.). Such timing control circuitry may also be used for the timing of refractory periods, blanking intervals, noise detection windows, evoked response windows, alert intervals, marker channel timing, and so on. The controller 112 can further include other dedicated circuitry and/or firmware/software components that assist in monitoring various conditions of the patient's heart and managing pacing therapies. For example, the controller 112 can include an arrhythmia detector, which can be similar to the arrhythmia detector 1134 discussed below with reference to FIG. 11.

The controller 112 and the pulse generator 116 may be configured to transmit event messages, via the electrodes 108, in a manner that does not inadvertently capture the heart in the chamber where LP 102a, 102b is located, such as when the associated chamber is not in a refractory state. In addition, an LP 102a, 102b that receives an event message may enter an "event refractory" state (or event blanking state) following receipt of the event message. The event refractory/blanking state may be set to extend for a determined period of time after receipt of an event message in order to avoid the receiving LP 102a, 102b from inadvertently sensing another signal as an event message that might otherwise cause retriggering. For example, the receiving LP 102a, 102b may detect a measurement pulse from another LP 102a, 102b or the programmer 109.

Since the conductive communication receivers 120, 122 (or alternatively, just a single conductive communication receiver) and the pulse generator 116 are used to perform conductive communication, these components can be considered parts of a conductive communication transceiver 124. The conductive communication transceiver 124 can alternatively include alternative and/or additional components that enable an LP 102, or other type of IMD, to transmit and receive conductive communication messages with another IMD and/or with an external device, such as the programmer 109. For an example, while the same pulse generator 116 can be used to produce pacing pulses (for use in pacing a patient's heart) and conductive communication pulses (for use in communicating with another IMD or an external device), it would also be possible that the conductive communication transceiver 124 can include its own dedicated pulse generator. For another example, as noted above, it would also be possible for the conductive communication transceiver 124 to include only a single receiver, rather than both receivers 120, 122. Other variations are also possible and within the scope of the embodiments described herein.

In accordance with certain embodiments herein, the programmer 109 may communicate over a programmer-to-LP channel, with LP 102a, 102b utilizing the same communication scheme. The external programmer 109 may listen to the event message transmitted between LP 102a, 102b and synchronize programmer to implant communication such that programmer 109 does not transmit communication messages 113 until after an i2i messaging sequence is completed.

The controller 120 is communicatively coupled to a memory 160, e.g., by a suitable data/address bus. The programmable operating parameters used by controller 120 are stored in memory 160 and used to customize the operation of LP 102a, 102b to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart within each respective tier of therapy.

The operating parameters of LP 102a, 102b may be non-invasively programmed into memory 160 through conductive communication transceiver 124 via communication link 113 with external device 109. Memory 160 may also store a table or other data structure that is dynamically updated over time to keep track of the types of i2i communication being used by the LP 102 and/or other IMDs with which LP 102a, 102b communicates. The memory 160 can also store a capture threshold and various types of templates. For example in accordance with certain embodiments of the present technology the memory 160 can store a polarization artifacts template and/or a capture plus polarization artifacts template, details of which are described below.

In some embodiments, each individual LP 102 can comprise a hermetic housing 110 configured for placement on or attachment to the inside or outside of a cardiac chamber and at least two leadless electrodes 108 proximal to the housing 110 and configured for bidirectional communication with at least one other device (e.g., an NV-ICD 106) within or outside the body.

FIG. 2 depicts a single LP 102 (e.g., the LP 102a or 102b) and shows the LP's functional elements substantially enclosed in a hermetic housing 110. The LP 102 has at least two electrodes 108 located within, on, or near the housing 110, for delivering pacing pulses to and sensing electrical activity from the muscle of the cardiac chamber, and for bidirectional communication with at least one other device within or outside the body. Hermetic feedthroughs 130, 131 conduct electrode signals through the housing 110. The housing 110 contains a primary battery 114 to supply power for pacing, sensing, and communication. The housing 110 also contains sense circuits 132 for sensing cardiac activity from the electrodes 108, preferably receivers 120, 122 for receiving information from at least one other device via the electrodes 108, and the pulse generator 116 for generating pacing pulses for delivery via the electrodes 108 and preferably also for transmitting information to at least one other device via the electrodes 108. The housing 110 can optionally contain circuits for monitoring device health, for example a battery current monitor 136 and a battery voltage monitor 138, and can contain circuits for controlling operations in a predetermined manner.

The electrodes 108 can be configured to communicate bidirectionally among the multiple leadless cardiac pacemakers and/or the implanted ICD 106 to coordinate pacing pulse delivery and optionally other therapeutic or diagnostic features using messages that identify an event at an individual pacemaker originating the message and a pacemaker receiving the message react as directed by the message depending on the origin of the message. An LP 102a, 102b that receives the event message reacts as directed by the event message depending on the message origin or location. In some embodiments or conditions, the two or more leadless electrodes 108 can be configured to communicate bidirectionally among the one or more leadless cardiac pacemakers 102 and/or the ICD 106 and transmit data including designated codes for events detected or created by an individual pacemaker. Individual pacemakers can be configured to issue a unique code corresponding to an event type and a location of the sending pacemaker.

In some embodiments, an individual LP 102a, 102b can be configured to deliver a pacing pulse with an event message encoded therein, with a code assigned according to pacemaker location and configured to transmit a message to one or more other leadless cardiac pacemakers via the event message coded pacing pulse. The pacemaker or pacemakers receiving the message are adapted to respond to the message in a predetermined manner depending on type and location of the event.

Moreover, information communicated on the incoming channel can also include an event message from another leadless cardiac pacemaker signifying that the other leadless cardiac pacemaker has sensed a heartbeat or has delivered a pacing pulse, and identifies the location of the other pacemaker. For example, LP 102b may receive and relay an event message from LP 102a to the programmer. Similarly, information communicated on the outgoing channel can also include a message to another leadless cardiac pacemaker or pacemakers, or to the ICD, that the sending leadless cardiac pacemaker has sensed a heartbeat or has delivered a pacing pulse at the location of the sending pacemaker.

Referring again to FIGS. 1 and 2, the cardiac pacing system 100 may comprise an ICD 106 in addition to one or more LPs 102a, 102b configured for implantation in electrical contact with a cardiac chamber and for performing cardiac rhythm management functions in combination with the implantable ICD 106. The implantable ICD 106 and the one or more LPs 102a, 102b configured for leadless intercommunication by information conduction through body tissue and/or wireless transmission between transmitters and receivers in accordance with the embodiments discussed herein.

In a further embodiment, a cardiac pacing system 100 comprises at least one LP 102a, 102b configured for implantation in electrical contact with a cardiac chamber and configured to perform cardiac pacing functions in combination with the co-implanted ICD 106. Each LP 102 comprise at least two leadless electrodes 108 configured for delivering cardiac pacing pulses, sensing evoked and/or natural cardiac electrical signals, and transmitting information to the co-implanted ICD 106.

As shown in the illustrative embodiments, an LP 102a, 102b can comprise two or more leadless electrodes 108 configured for delivering cardiac pacing pulses, sensing evoked and/or natural cardiac electrical signals, and bidirectionally communicating with the co-implanted ICD 106.

Each LP 102a, 102b can be configured for operation in a respective particular location and to have a respective particular functionality at manufacture and/or by programming by an external programmer. Bidirectional communication among the multiple leadless cardiac pacemakers can be arranged to communicate notification of a sensed heartbeat or delivered pacing pulse event and encoding type and location of the event to another implanted pacemaker or pacemakers. The LP 102a, 102b receiving the communication decode the information and respond depending on location of the receiving pacemaker and predetermined system functionality.

Also shown in FIG. 2, the primary battery 114 has positive terminal 140 and negative terminal 142. Current from the positive terminal 140 of primary battery 114 flows through an optional shunt 144 to an optional regulator circuit 146 to create a positive voltage supply 148 suitable for powering the remaining circuitry of the pacemaker 102. The optional shunt 144 enables the optional battery current monitor 136 to provide the controller 112 with an indication of battery current drain and indirectly of device health. The illustrative power supply can be a primary battery 114.

Still referring to FIG. 2, the LP 102 is shown as including an optional temperature sensor 152. The temperature sensor 152 can be any one of various different types of well-known temperature sensors, or can be a future developed temperature sensor. For one example, the temperature sensor 152 can be a thermistor, a thermocouple, a resistance thermometer, or a silicon bandgap temperature sensor, but is not limited thereto. Regardless of how the temperature sensor 152 is implemented, it is preferably that the temperature sensed by the temperature sensor 152 is provided to the controller 112 as a digital signal indicative of the blood temperature of the patient within which the LP is implanted. The temperature sensor 152 can be hermetically sealed within the housing 110, but that need not be the case. The temperature sensor 152 can be used in various manners. For example, the temperature sensor 152 can be used to detect an activity level of the patient to adjust a pacing rate, i.e., for use in rate responsive pacing. When a person starts to exercise their core body temperature initially dips, and then after exercising for a prolonged period of time the person's core body temperature will eventually rise. Thereafter, when the person stops exercising their core body temperature will return to its baseline. Accordingly, the controller 112 can be configured to detect an activity level of a patient based on core blood temperature measurements obtained using the temperature sensor 152.

Referring to FIG. 2, the LP 102 is also shown as including an optional accelerometer 154 which can be hermetically contained within the housing 110. The accelerometer 154 can be any one of various different types of well-known accelerometers, or can be a future developed accelerometer. For one example, the accelerometer 154 can be or include, e.g., a MEMS (micro-electromechanical system) multi-axis accelerometer of the type exploiting capacitive or optical cantilever beam techniques, or a piezoelectric accelerometer that employs the piezoelectric effect of certain materials to measure dynamic changes in mechanical variables. For example, the accelerometer 154 can be used to detect an activity level of the patient to adjust a pacing rate, i.e., for use in rate responsive pacing. It would also be possible to use outputs of both the accelerometer 154 and the temperature sensor 152 to monitor the activity level of a patient. Alternatively, or additionally, a patient's activity level can be monitored based on their heart rate, as detected from an IEGM sensed using the electrodes 108, and/or sensed using a plethysmography signal obtained using a plethysmography sensor (not shown) or a heart sound sensor (not shown), but not limited thereto. One or more signals produced and output by the accelerometer 154 may be analyzed with respect to frequency content, energy, duration, amplitude and/or other characteristics. Such signals may or may not be amplified and/or filtered prior to being analyzed. For example, filtering may be performed using lowpass, highpass and/or bandpass filters. The signals output by the accelerometer 154 can be analog signals, which can be analyzed in the analog domain, or can be converted to digital signals (by an analog-to-digital converter) and analyzed in the digital domain. Alternatively, the signals output by the accelerometer 154 can already be in the digital domain. The one or more signals output by the accelerometer 154 can be analyzed by the controller 112 and/or other circuitry. In certain embodiments, the accelerometer 154 is packaged along with an integrated circuit (IC) that is designed to analyze the signal(s) it generates. In such embodiments, one or more outputs of the packaged sensor/IC can be an indication of acceleration along one or more axes. In other embodiments, the accelerometer 154 can be packaged along with an IC that performs signal conditioning (e.g., amplification and/or filtering), performs analog-to-digital conversions, and stores digital data (indicative of the sensor output) in memory (e.g., RAM, which may or may not be within the same package). In such embodiments, the controller 112 or other circuitry can read the digital data from the memory and analyze the digital data. Other variations are also possible, and within the scope of embodiments of the present technology. In accordance with certain embodiments of the present technology, described in additional detail below, a sensor signal produced by the accelerometer 154 of an LP 102 implanted in or on a cardiac chamber can be used to detect mechanical cardiac activity associated with another cardiac chamber.

In various embodiments, LP 102a, 102b can manage power consumption to draw limited power from the battery 114, thereby reducing device volume. Each circuit in the LP 102a, 102b can be designed to avoid large peak currents. For example, cardiac pacing can be achieved by discharging a tank capacitor (not shown) across the pacing electrodes 108. Recharging of the tank capacitor is typically controlled by a charge pump circuit. In a particular embodiment, the charge pump circuit is throttled to recharge the tank capacitor at constant power from the battery 114.

In some embodiments, the controller 112 in an LP 102 can access signals on the electrodes 108 and can examine output pulse duration from another LP 102 for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller 112 can examine output pulse waveform from another leadless cardiac pacemaker for usage as a signature for determining triggering information validity and, for a signature arriving within predetermined limits, activating delivery of a pacing pulse following a predetermined delay of zero or more milliseconds.

FIG. 3 shows an example form factor of the LPs 102a, 102b. Each LP 102a, 102b can include a hermetic housing 202 (e.g., 110 in FIG. 2) with electrodes 108a and 108b disposed thereon. As shown, electrode 108a can be separated from but surrounded partially by a fixation mechanism 205, and the electrode 108b can be disposed on the housing 202. The fixation mechanism 205 can be a fixation helix, a plurality of hooks, barbs, or other attaching features configured to attach the pacemaker to tissue, such as heart tissue. The electrodes 108a and 108b are examples of the electrodes 108 shown in and discussed above with reference to FIG. 2.

The housing 202 can also include an electronics compartment 210 within the housing that contains the electronic components necessary for operation of the pacemaker, including, e.g., a pulse generator, transceiver, a battery, and a processor for operation. The hermetic housing 202 can be adapted to be implanted on or in a human heart, and can be cylindrically shaped, rectangular, spherical, or any other appropriate shapes, for example.

The housing 202 can comprise a conductive, biocompatible, inert, and anodically safe material such as titanium, 316L stainless steel, or other similar materials. The housing 202 can further comprise an insulator disposed on the conductive material to separate electrodes 108a and 108b. The insulator can be an insulative coating on a portion of the housing 202 between the electrodes 108a, 108b, and can comprise materials such as silicone, polyurethane, parylene, or another biocompatible electrical insulator commonly used for implantable medical devices. In the embodiment of FIG. 2, a single insulator 208 is disposed along the portion of the housing 202 between electrodes 108a and 108b. In some embodiments, the housing 202 itself can comprise an insulator instead of a conductor, such as an alumina ceramic or other similar materials, and the electrodes 108a, 108b can be disposed upon the housing 202.

As shown in FIG. 3, the LP 102a, 102b can further include a header assembly 212 to isolate the electrodes 108a and 108b. The header assembly 212 can be made from PEEK, tecothane or another biocompatible plastic, and can contain a ceramic to metal feedthrough, a glass to metal feedthrough, or other appropriate feedthrough insulator as known in the art.

The electrodes 108a and 108b can comprise pace/sense electrodes, or return electrodes. A low-polarization coating can be applied to the electrodes 108a, 108b, such as sintered platinum, platinum-iridium, iridium, iridium-oxide, titanium-nitride, carbon, or other materials commonly used to reduce polarization effects, for example. In FIG. 2, electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A helical fixation mechanism 205, also referred to as (helical) fixation device 205 herein, can enable insertion of the LP 102a, 102b endocardially or epicardially through a guiding catheter. A torqueable catheter can be used to rotate the housing 202 and force the fixation device 205 into heart tissue, thus affixing the fixation device 205 (and also the electrode 108a in FIG. 2) into contact with stimulable tissue. Electrode 108b can serve as an indifferent electrode for sensing and pacing. The fixation mechanism 205 may be coated partially or in full for electrical insulation, and a steroid-eluting matrix may be included on or near the LP 102a, 102b to minimize fibrotic reaction, as is known in conventional pacing electrode-leads. Implant-to-Implant Event Messaging

LPs 102a and 102b can utilize implant-to-implant (i2i) communication through event messages to coordinate operation with one another in various manners. The terms i2i communication, i2i event messages, and i2i event markers are used interchangeably herein to refer to event related messages and IMD/IMD operation related messages transmitted from an implanted device and directed to another implanted device (although external devices, e.g., a programmer, may also receive i2i event messages). In certain embodiments, LP 102a and LP 102b operate as two independent leadless pacers maintaining beat-to-beat dual-chamber functionality via a "Master/Slave" operational configuration. For descriptive purposes, the ventricular LP 102b shall often be referred to as "vLP" and the atrial LP 102a shall often be referred to as "aLP". The LP 102 that is designated as the master device (e.g., vLP) may implement all or most dual-chamber diagnostic and therapy determination algorithms. For purposes of the following illustration, it is assumed that the vLP is a "master" device, while the aLP is a "slave" device. Alternatively, the aLP may be designated as the master device, while the vLP may be designated as the slave device. The master device orchestrates most or all decision-making and timing determinations (including, for example, rate-response changes).

In accordance with certain examples, exemplary methods are provided for coordinating operation between first and second leadless pacemakers (LPs) configured to be implanted entirely within (or on) first and second chambers of the heart. A method transmits an event marker through conductive communication through electrodes located along a housing of the first LP, the event marker indicative of one of a local paced or sensed event. The method detects, over a sensing channel, the event marker at the second LP. The method identifies the event marker at the second LP based on a predetermined pattern configured to indicate that an event of interest has occurred in a remote chamber. In response to the identifying operation, the method initiates a related action in the second LP. In certain embodiments, event messages and/or other types of i2i messages can be alternatively, or additionally, transmitted using RF communication, as will be described in more detail below.

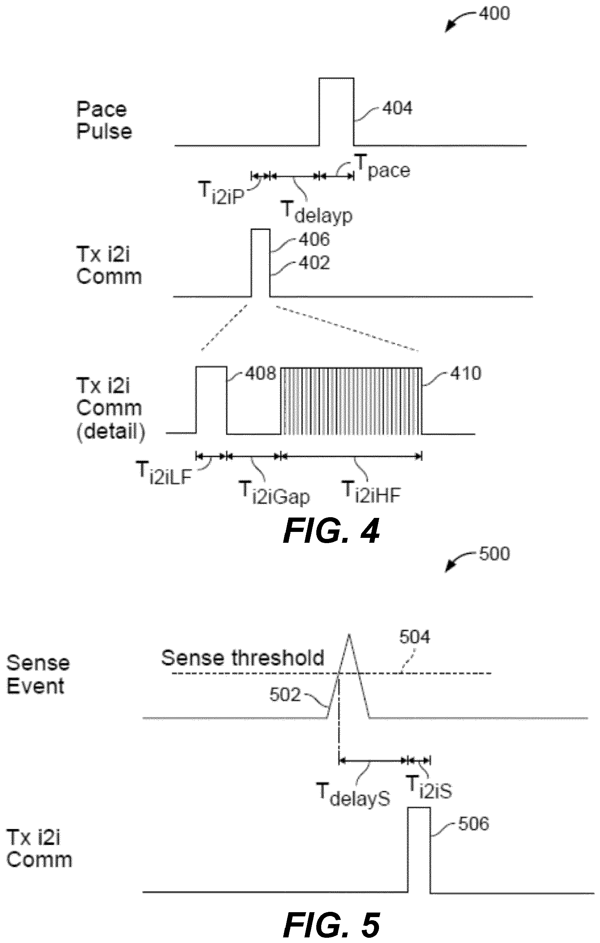

FIG. 4 is a timing diagram 400 demonstrating one example of an i2i conductive communication for a paced event. The i2i conductive communication may be transmitted, for example, from LP 102a to LP 102b. As shown in FIG. 4, in this embodiment, an i2i transmission 402 is sent prior to delivery of a pace pulse 404 by the transmitting LP (e.g., LP 102a). This enables the receiving LP (e.g., LP 102b) to prepare for the remote delivery of the pace pulse. The i2i transmission 402 includes an envelope 406 that may include one or more individual pulses. For example, in this embodiment, envelope 406 includes a low frequency pulse 408 followed by a high frequency pulse train 410. Low frequency pulse 408 lasts for a period Ti2iLF, and high frequency pulse train 410 lasts for a period Ti2iHF. The end of the low frequency pulse 408 and the beginning of the high frequency pulse train 410 are separated by a gap period, Ti2iGap. In alternative embodiments, rather than transmitting the envelope 406 prior to the pacing pulse 404, the envelope 406 can be transmitted during a refractory period that follows the delivery of the pacing pulse.

As shown in FIG. 4, the i2i transmission 402 lasts for a period Ti2iP, and pace pulse 404 lasts for a period Tpace. The end of i2i transmission 402 and the beginning of pace pulse 404 are separated by a delay period, TdelayP. The delay period may be, for example, between approximately 0.0 and 10.0 milliseconds (ms), particularly between approximately 0.1 ms and 2.0 ms, and more particularly approximately 1.0 ms. The term approximately, as used herein, means +/- 10% of a specified value.

FIG. 5 is a timing diagram 500 demonstrating one example of an i2i communication for a sensed event. The i2i communication may be transmitted, for example, from LP 102a to LP 102b. As shown in FIG. 5, in this embodiment, the transmitting LP (e.g., LP 102a detects the sensed event when a sensed intrinsic activation 502 crosses a sense threshold 504. A predetermined delay period, TdelayS, after the detection, the transmitting LP transmits an i2i transmission 506 that lasts a predetermined period Ti2iS. The delay period may be, for example, between approximately 0.0 and 10.0 milliseconds (ms), particularly between approximately 0.1 ms and 2.0 ms, and more particularly approximately 1.0 ms.

As with i2i transmission 402, i2i transmission 506 may include an envelope that may include one or more individual pulses. For example, similar to envelope 406, the envelope of i2i transmission 506 may include a low frequency pulse followed by a high frequency pulse train. In certain embodiments, the i2i transmission 506 is transmitted during a refractory period that follows the sensed event.

Optionally, wherein the first LP is located in an atrium and the second LP is located in a ventricle, the first LP produces an AS/AP event marker to indicate that an atrial sensed (AS) event or atrial paced (AP) event has occurred or will occur in the immediate future. For example, the AS and AP event markers may be transmitted following the corresponding AS or AP event. Alternatively, the first LP may transmit the AP event marker slightly prior to delivering an atrial pacing pulse. Alternatively, wherein the first LP is located in an atrium and the second LP is located in a ventricle, the second LP initiates an atrioventricular (AV) interval after receiving an AS or AP event marker from the first LP; and initiates a post atrial ventricular blanking (PAVB) interval after receiving an AP event marker from the first LP.

Optionally, the first and second LPs may operate in a "pure" master/slave relation, where the master LP delivers "command" markers in addition to or in place of "event" markers. A command marker directs the slave LP to perform an action such as to deliver a pacing pulse and the like. For example, when a slave LP is located in an atrium and a master LP is located in a ventricle, in a pure master/slave relation, the slave LP delivers an immediate pacing pulse to the atrium when receiving an AP command marker from the master LP.

In accordance with some embodiments, communication and synchronization between the aLP and vLP is implemented via conducted communication of markers/commands in the event messages (per i2i communication protocol). As explained above, conducted communication represents event messages transmitted from the sensing/pacing electrodes at frequencies outside the RF (e.g., Wi-Fi or BLE) frequency range. Alternatively, the event messages may be conveyed over communication channels operating in the RF frequency range. The figures and corresponding description below illustrate non-limiting examples of markers that may be transmitted in event messages. The figures and corresponding description below also include the description of the markers and examples of results that occur in the LP that receives the event message. Table 1 represents example event markers sent from the aLP to the vLP, while Table 2 represents example event markers sent from the vLP to the aLP. In the master/slave configuration, AS event markers are sent from the aLP each time that an atrial event is sensed outside of the post ventricular atrial refractory period (PVARP) interval or some other alternatively-defined atrial refractory period. The AP event markers are sent from the aLP each time that the aLP delivers a pacing pulse in the atrium. The aLP may restrict transmission of AS markers, whereby the aLP transmits AS event markers when atrial events are sensed both outside of the PVAB interval and outside the post ventricular atrial refractory period (PVARP) or some other alternatively-defined atrial refractory period. Alternatively, the aLP may not restrict transmission of AS event markers based on the PVARP, but instead transmit the AS event marker every time an atrial event is sensed.

**Table 1**

| "A2V" Markers / Commands (*i.e.,* from aLP to vLP) | | |
|---|---|---|
| *Marker* | *Description* | *Result in vLP* |
| AS | Notification of a sensed event in atrium (if not in PVAB or PVARP) | • Initiate AV interval (if not in PVAB or PVARP) |
| AP | Notification of a paced event in atrium | • Initiate PAVB |
| | | • Initiate AV interval (if not in PVARP) |

As shown in Table 1, when an aLP transmits an event message that includes an AS event marker (indicating that the aLP sensed an intrinsic atrial event), the vLP initiates an AV interval timer. If the aLP transmits an AS event marker for all sensed events, then the vLP would preferably first determine that a PVAB or PVARP interval is not active before initiating an AV interval timer. If however the aLP transmits an AS event marker only when an intrinsic signal is sensed outside of a PVAB or PVARP interval, then the vLP could initiate the AV interval timer upon receiving an AS event marker without first checking the PVAB or PVARP status. When the aLP transmits an AP event marker (indicating that the aLP delivered or is about to deliver a pace pulse to the atrium), the vLP initiates a PVAB timer and an AV interval time. The vLP may also blank its sense amplifiers to prevent possible crosstalk sensing of the remote pace pulse delivered by the aLP.

**Table 2**

| "V2A" Markers / Commands (*i.e.,* from vLP to aLP) | | |
|---|---|---|
| *Marker* | *Description* | *Result in aLP* |
| VS | Notification of a sensed event in ventricle | • Initiate PVARP |
| VP | Notification of a paced event in ventricle | • Initiate PVAB |
| | | • Initiate PVARP |
| AP | Command to deliver immediate pace pulse in atrium | • Deliver immediate pace pulse to atrium |

As shown in Table 2, when the vLP senses a ventricular event, the vLP transmits an event message including a VS event marker, in response to which the aLP may initiate a PVARP interval timer. When the vLP delivers or is about to deliver a pace pulse in the ventricle, the vLP transmits VP event marker. When the aLP receives the VP event marker, the aLP initiates the PVAB interval timer and also the PVARP interval timer. The aLP may also blank its sense amplifiers to prevent possible crosstalk sensing of the remote pace pulse delivered by the vLP. The vLP may also transmit an event message containing an AP command marker to command the aLP to deliver an immediate pacing pulse in the atrium upon receipt of the command without delay.

The foregoing event markers are examples of a subset of markers that may be used to enable the aLP and vLP to maintain full dual chamber functionality. In one embodiment, the vLP may perform all dual-chamber algorithms, while the aLP may perform atrial-based hardware-related functions, such as PVAB, implemented locally within the aLP. In this embodiment, the aLP is effectively treated as a remote 'wireless' atrial pace/sense electrode. In another embodiment, the vLP may perform most but not all dual-chamber algorithms, while the aLP may perform a subset of diagnostic and therapeutic algorithms. In an alternative embodiment, vLP and aLP may equally perform diagnostic and therapeutic algorithms. In certain embodiments, decision responsibilities may be partitioned separately to one of the aLP or vLP. In other embodiments, decision responsibilities may involve joint inputs and responsibilities.

Messages that are transmitted between LPs (e.g., the aLP and the vLP) can be referred to herein generally as i2i messages, since they are implant-to-implant messages. As noted above, such messages can include event markers that enable one LP to inform the other LP of a paced event or a sensed event. For example, in certain embodiments, whenever the aLP 102a senses an atrial event or paces the right atrium, the aLP will transmit an i2i message to the vLP 102b to inform the vLP of the sensed or paced event in the atrium. In response to receiving such an i2i message, the vLP 102b may start one or more timers that enable the vLP to sense or pace in the right ventricle. Similarly, the vLP may transmit an i2i message to the aLP 102a whenever the vLP senses a ventricular event or paces the right ventricle.

The i2i messages that are sent between LPs may be relatively short messages that simply allow a first LP to inform a second LP of an event that was sensed by the first LP or caused (paced) by the first LP, and vice versa. Such i2i messages can be referred to herein as event marker i2i messages, or more succinctly as event i2i messages. The i2i messages that are sent between LPs, in certain instances, can be extended i2i messages that include (in addition to an event marker) an extension. In certain embodiments, an extended i2i message includes an event marker (e.g., 9 bits), followed by an extension indicator (e.g., 2 bits), followed by an extended message payload portion (e.g., 17 bits), followed by a cyclic redundancy check (CRC) code (e.g., 6 bits) or some other type of error detection and correction code.

### Capture Management

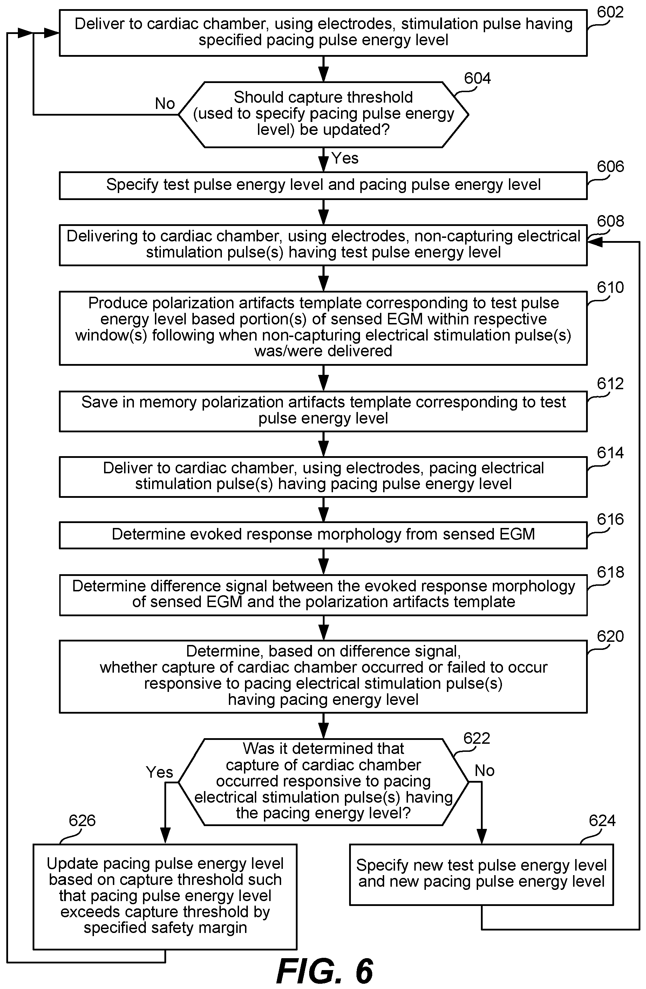

The high level flow diagram of FIG. 6 will now be used to summarize certain embodiments of the present disclosure, that provide for capture management in manners that compensate for polarization artifacts and may provide for efficient use of limited memory available in IMDs, and in particular LPs. Such embodiments can be performed, e.g., by an LP implanted in or on a cardiac chamber of a heart (e.g., in or on an atrial chamber), wherein the LP includes electrodes and memory. In certain embodiments, the LP uses the same electrodes for delivering electrical stimulation pulses to the cardiac chamber and for sensing an EGM indicative of electrical activity of the heart.

Referring to FIG. 6, step 602 involves the LP delivering a stimulation pulse having a specified pacing pulse energy level to the cardiac chamber in or on which the LP is implanted. Initially, the specified pacing pulse energy level used at instances of step 602 may been specified by default, or based on a capture threshold that had been previously determined for the patient. For example, the specified pacing pulse energy level can be specified based on a previously determined capture threshold to which a safety margin (aka a safety factor) has been applied. Pacing pulse energy (E), which can also be referred to as stimulation energy, is equal to a pacing voltage^2 (V, squared) multiplied by a pulse duration (t) divided by a pacing impedance (R), i.e., E = (V² × t) / R. In certain embodiments, the pulse duration and pacing impedance remain the same and the pacing pulse energy is adjusted by only adjusting the pacing voltage. In certain such embodiments, once a capture threshold is determined, a safety margin can be applied by multiplying the pacing voltage (corresponding to the capture threshold) by a multiplier (larger than one), or the safety margin can be applied by adding a voltage to the pacing voltage (corresponding to the capture threshold). For some examples, applying a multiplier safety marker of 1.4 to the pacing voltage (corresponding to the capture threshold) effectively doubles the stimulation energy (since 1.4^2 ≈ 2); applying a multiplier safety margin of 1.7 to the pacing voltage (corresponding to the capture threshold) effectively triples the stimulation energy (since 1.7^2 ≈ 3); and applying a multiplier safety margin of 2 (i.e., doubling the pacing voltage corresponding to the capture threshold) effectively quadruples the stimulation energy (since 2^2 = 4). Where the safety margin is a multiplier, it is larger than one can, in an embodiment, be within a specified range from 1.2 to 2.0, but is not limited thereto. Where the safety margin is additive, it can be within a specified range, e.g., from 1.0 V to 3.0 V, but is not limited thereto. A safety margin can alternatively, or additionally, be applied by increasing the pulse width of stimulation pulses, as is known in the art. Other ways of specifying a pacing pulse energy (aka stimulation energy) based on a capture threshold and a safety margin are also possible and within the scope of the embodiments described herein.

For much of the following discussion it is assumed that the steps described with reference to FIG. 6 are performed by an aLP implanted in or on an atrial chamber of a patient's heart, and it is assumed that a vLP is also implanted in or on a ventricular chamber of the patient's heart, and that the aLP and vLP are configured to communicate with one another using i2i messages. Although it is also within the scope of the present disclosure for the steps described with reference to FIG. 6 to be performed by a vLP implanted in or on a ventricular chamber of a patient's heart. Collectively, the aLP and the vLP can be capable of supporting coordinated dual chamber operating modes, such as DDD, VDD, DDI, VDI, or DOO. Individually, the aLP can be capable of providing single chamber operating modes, e.g., AAI, or AAO, but not limited thereto. Individually, the vLP can be capable of providing single chamber operating modes, e.g., VVI, or VVO, but not limited to thereto. Assuming that step 602 is performed by an aLP, the specific time at which the aLP delivers a pacing pulse to the atrial chamber at an instance of step 602 can depend on many factors, including the specific operating mode being used, a specific programmed delay and interval settings (e.g., settings for AV delay, AA interval, and/or the like), whether the pacing is rate responsive, and/or the like.

Still referring to FIG. 6, at step 604 there is a determination of whether a capture threshold should be updated, wherein the capture threshold is used by the LP to specify a pacing pulse energy level. For example, the aLP can be configured to periodically update its capture threshold once every 8 hours, once per day (or once per other period of time), and/or in response to a triggering event. Assuming the aLP is configured to periodically update its capture threshold once per period of time, at step 604 there can be a determination of whether a specified period of time (e.g., 8 hours, 24 hours, or the like) has elapsed since the capture threshold was last updated. Additionally, or alternatively, at step 604 there can be a determination whether a triggering event occurred, in response to which the capture threshold should be updated. The triggering event can be, e.g., capture failing to be achieved in a specified number of cardiac cycles, a person in which the LP is implanted changing their posture, or a person in which the LP is implanted changing their activity level, but is not limited thereto.

If the answer to the determination at step 604 is No, then flow returns to step 602, and the LP delivers another stimulation pulse having the specified pacing pulse energy level to the cardiac chamber in or on which the LP is implanted. When the answer to the determination at step 604 is Yes, flow goes to step 606.

At step 606 a test pulse energy level is specified, and a pacing pulse energy level is specified. The test pulse energy level, after being specified, is used at step 608 discussed below; and the pacing pulse energy level, after being specified, is used at step 614 discussed below. While shown as a single step in FIG. 6, step 606 can be shown as two separate steps, one of which involves specifying the test pulse energy level, and another of which involves specifying the pacing pulse energy level.

Step 608 involves the LP delivering to the cardiac chamber, using its electrodes and the pulse generator, one or more non-capturing electrical stimulation pulses having the test pulse energy level (that had been specified at step 606), and step 610 involves producing a polarization artifacts template corresponding to the test pulse energy level based on one or more portions of the sensed EGM within one or more respective windows following when the one or more non-capturing electrical stimulation pulses was/were delivered at step 608. A reason that stimulation pulses delivered at step 608 should be non-capturing, i.e., should fail to achieve capture, is so that the template produced at step 610 is entirely or primarily representative of polarization artifacts, and not at all representative of a depolarization that corresponds to capture. In accordance with certain embodiments, in order to ensure that capture is not achieved at step 608, the one or more non-capturing electrical stimulation pulses (having the test pulse energy level) are preferably delivered during one or more refractory periods corresponding to one or more windows following one or more depolarizations of the cardiac chamber. Alternatively, or in addition, the test pulse energy level can be specified at step 606 such that it is below the most recently determined capture threshold, in which case it would be likely that the electrical stimulation pulse(s) delivered at step 608 would fail to achieve capture.

In accordance with certain embodiments, during one cardiac cycle, one non-capturing electrical stimulation pulse having the test pulse energy level is delivered at step 608, and the polarization artifacts template corresponding to the test pulse energy level is produced at step 610 based a portion of a sensed EGM within one window (e.g., one 50 msec window) following when the single non-capturing electrical stimulation pulse was delivered at step 608 (e.g., the 50 msec window can begin 15 msec after the non-capturing electrical stimulation pulse is delivered). In accordance with other embodiments, during each of a plurality of cardiac cycles, a non-capturing electrical stimulation pulse having the test pulse energy level is delivered at step 608, and the polarization artifacts template corresponding to the test pulse energy level is produced at step 610 based portions of a sensed EGM within respective windows (e.g., respective 50 msec windows) following when non-capturing electrical stimulation pulses were delivered at step 608 (e.g., each of the 50 msec windows can begin 15 msec after each of the non-capturing electrical stimulation pulses were delivered). For example, at step 610, portions of the sensed EGM, within respective windows following when the non-capturing electrical stimulation pulses were delivered at step 608, can be averaged (e.g., ensemble averaged) to produce the polarization artifacts template corresponding to the test pulse energy level. Step 612 involves saving, within a portion of the memory of the LP, the polarization artifacts template corresponding to the test pulse energy level, which template was determined at step 610. In accordance with certain embodiments, the polarization artifacts template corresponds to an expected morphology of a sensed EGM responsive to the delivery of one of the non-capturing electrical stimulation pulses having the test pulse energy level.

Still referring to FIG. 6, step 614 involves the LP delivering to the cardiac chamber (using its electrodes and pulse generator) one or more pacing electrical stimulation pulses having the pacing pulse energy level (that had been specified at step 606), step 616 involves determining an evoked response morphology, and step 618 involves determining a difference signal between the evoked response morphology of the sensed EGM (determined at step 616) and the polarization artifacts template (produced at step 610).

In accordance with certain embodiments, during one cardiac cycle, one pacing electrical stimulation pulse having the pacing pulse energy level is delivered at step 614, and the evoked response morphology (corresponding to the pacing pulse energy level) is produced at step 616 based a portion of a sensed EGM within one window (e.g., a 50 msec window) following when the single pacing electrical stimulation pulse was delivered at step 614 (e.g., the 50 msec window can begin 15 msec after the single pacing electrical stimulation pulse is delivered). In accordance with other embodiments, during each of a plurality of cardiac cycles, a pacing electrical stimulation pulse having the pacing pulse energy level is delivered at step 614, and the evoked response morphology corresponding to the pacing pulse energy level is produced at step 616 based portions of a sensed EGM within respective windows following when pacing electrical stimulation pulses were delivered at step 614. For example, at step 616, portions of the sensed EGM, within respective windows (e.g., respective 50 msec window) following (e.g., 15 msec after) when the pacing electrical stimulation pulses were delivered at step 614, can be averaged (e.g., ensemble averaged) to determine the evoked response morphology corresponding to the pacing pulse energy level. In accordance with certain embodiments, the evoked response morphology determined at step 616 is temporarily stored in a portion of the memory (e.g., in buffer memory) of the LP before it is used in the subtraction performed at step 618. Depending upon the specific implementation, the aforementioned windows can be longer or shorter than 50 msec, e.g., such windows can have a duration in the range of about 25 msec to 75 msec, but is not limited thereto. Further, such windows need not start at exactly 15 msec following when the pacing electrical stimulation pulse(s) was/were delivered at step 614. For example, such windows can more generally start anywhere within the range of about 2 msec to about 30 msec following when the pacing electrical stimulation pulse(s) was/were delivered at step 614, but is not limited thereto. In other words, in certain embodiments the aforementioned windows can start anywhere within the range of about 2 msec to about 30 msec (e.g., 15 msec) following when the pacing electrical stimulation pulse(s) was/were delivered at step 614, and such windows can have a duration in the range of about 25 msec to 75 msec (e.g., 50 msec), but are not limited thereto.

Still referring to FIG. 6, at step 620 there is a determination, based on the difference signal (that had been determined at step 618), whether capture of the cardiac chamber occurred or failed to occur responsive to the pacing electrical stimulation pulse(s) having the pacing pulse energy level (that had been delivered at step 614). If the difference signal has a morphology that is substantially flat, that means the polarization artifacts template and the evoked response morphology have substantially the same morphologies as one another, which is indicative pacing electrical stimulation pulse(s) having the pacing pulse energy level (that had been delivered at step 614) failing to achieve capture. On the other hand, if the difference signal has a morphology that is not substantially flat and includes a recognizable and substantial inflection point, that means the polarization artifacts template and the evoked response morphology have substantially different morphologies than another, which is indicative pacing electrical stimulation pulse(s) having the pacing pulse energy level (that had been delivered at step 614) successfully achieving capture.

At step 622 there is a determination of whether, at step 620, it was determined that capture of the cardiac chamber occurred responsive to the pacing electrical stimulation pulse(s) having the pacing pulse energy level (that had been delivered at step 614). If the answer to the determination at step 622 is No, meaning that pacing electrical stimulation pulse(s) having the pacing pulse energy level failed to achieve capture, then flow goes to step 624. At step 624, a new test pulse energy level is specified and a new pacing pulse energy level is specified by the controller of the LP. While steps 620 and 622 are shown as two separate steps, they could have been shown as a single step.

After step 624, flow returns to step 608, at which the cardiac chamber is paced (using its electrodes) using the new test pulse energy level (specified at the most recent instance of step 624), which results a new polarization artifacts template being produced (at the next instance of step 610) and saved (at the next instance of step 612). In accordance with certain embodiments, the new polarization artifacts template (produced at step 610) is saved (at the present instance of step 612) in the same portion of the memory by overwriting the polarization artifacts template (that had been saved in the preceding instance of step 612). Such features involving the overwriting of the previously stored polarization artifacts template with the newly produced polarization artifacts template provide for efficient use of the limited memory that is available in the LP due to the relatively small size of the LP. For example, such embodiments provide for significantly more efficient use of the memory of the LP than would be the case if a separate polarization artifacts template were stored in the memory for each of a plurality of different possible pacing pulse energy levels (in which case, the memory would contemporaneously store a plurality of separate polarization artifacts template).

Still referring to FIG. 6, when the answer to the determination at step 622 is Yes, flow goes to step 626. At step 626 the pacing pulse energy level (to be used during further pacing of the cardiac chamber) is updated based on the most recently (i.e., last) determined capture threshold (which corresponds to the pacing pulse energy level that caused the answer to step 622 to be Yes). In other words, once there is a determination at step 622 that capture successfully occurred, then the pacing pulse energy level that caused the capture is considered to be the new capture threshold. Then, at step 622, the updated pacing pulse energy is determined that exceeds the new capture threshold by the safety margin (aka safety factor). As noted above, the safety margin can be a multiplier or additive. For example, once a capture threshold is determined, a safety margin can be applied by multiplying the pacing voltage (corresponding to the capture threshold) by a multiplier, or the safety margin can be applied by adding a voltage to the pacing voltage (corresponding to the capture threshold). A safety margin can alternatively, or additionally, be applied by increasing the pulse width of stimulation pulses, as is known in the art. Additional details of how a safety margin can be applied, after a capture threshold is determined, were described above when initially discussing step 602. Other ways of specifying an updated pacing pulse energy (at step 626) based on a capture threshold (determined after the answer to the determination at step 622 is Yes) and a safety margin are also possible and within the scope of the embodiments described herein.

After the pacing pulse energy level is updated at step 626, flow returns to step 602, at which a stimulation pulse having the specified (and more specifically updated) pacing pulse energy level is delivered to the cardiac chamber by the LP, using electrodes of the LP. The updated pacing pulse energy level continues to be used to pace the cardiac chamber until there is a determination at step 604 that the capture threshold, which had been used to specify the pacing pulse energy level, should again be updated.

Still referring to FIG. 6, it should be appreciated from the flow diagram shown therein that when the answer to the determination at step 622 is No (i.e., in response to determining that capture of the cardiac chamber failed to occur responsive to the one or more pacing electrical stimulation pulses having a specific pacing pulse energy level), each of steps 608 through 622 are repeated, such that a new test pulse energy level is used at the repeated step 608, such that a new polarization artifacts template corresponding to the new test pulse energy level is produced at the repeated step 610 and is saved at the repeated step 612 in the same portion of the memory by overwriting the polarization artifacts template that had been saved in a preceding instance of step 612, such that the new pacing pulse energy level is used at the repeated step 614, and such that the determination at step 620 (and step 622) is whether capture of the cardiac chamber occurred or failed to occur responsive to one or more pacing electrical stimulation pulses having the new pacing pulse energy level.

As noted above, in certain embodiments, at step 608, the one or more non-capturing electrical stimulation pulses (having the test pulse energy level) are delivered during one or more refractory periods corresponding to one or more windows following one or more depolarizations of the cardiac chamber, and are used to produce a corresponding polarization artifacts template at step 610, which is saved in memory at step 612. In certain such embodiments, the pacing pulse energy level of the one or more pacing electrical stimulation pulses(s), delivered at step 614, is the same as the test pulse energy level. This increases the probability that the polarization artifacts template corresponds to the pacing pulse energy level that is being tested to determine whether its magnitude is sufficient to successfully cause capture. Such an embodiment can be performed by, at step 606, specifying that test pulse energy level and the pacing pulse energy level are the same as one another.

In alternative embodiments, the test pulse energy level of the non-capturing electrical stimulation pulse(s) delivered during refractory period(s) at step 608 (and used to produce a corresponding polarization artifacts template at step 610, and saved in memory at step 612) can be different than, e.g., slightly less than or slightly greater than, the pacing pulse energy level of the one or more pacing electrical stimulation pulses(s) delivered at step 614. This is because if a difference between the test pulse energy level (of the non-capturing electrical stimulation pulse(s), delivered during refractory period(s) at step 608) and the pacing pulse energy level (of the one or more pacing electrical stimulation pulses(s) delivered at step 614) is sufficiently small (e.g., they are within 10% of one another), the polarization artifacts template (produced at step 610, and saved at step 612) should be close enough to a template that corresponds exactly to the pacing pulse energy level that is being tested, such that the template can be used to determine a difference signal (at step 618) based upon which there can be a determination of whether or not capture successfully occurred (at step 620).

As was also noted above, in other embodiments, at step 606 the test pulse energy level can be specified (and then used at step 608) such that it is sufficiently below the most recently (i.e., last) determined capture threshold, such that the electrical stimulation pulse(s) delivered at step 608 should fail to achieve capture, and thereby can used to produce a polarization artifacts template at step 610, which is saved at step 612. In such embodiments, the pacing pulse energy level specified at step 606 (and then used at step 614) can be a specified delta above the test pulse energy level specified at step 606, wherein the delta can be a multiplier (e.g., the delta multiplier can be in the range of 1.01 to 1.10) or can be additive. Accordingly, in such embodiments, when performance of steps 606 through 620 (during one loop through the flow diagram) results in a determination at step 620 that capture failed to occur, the new test pulse energy level and the new pacing pulse energy level can be specified at step 624 by increasing both the test pulse energy level and the pacing pulse energy level that had just been used (at the instances of steps 608 and 614 that were just performed). Such increases can be achieved by increasing the amplitude and/or pulse width of each of the test pulse energy level and the pacing pulse energy level.

It is also possible to combine the above-described embodiments, i.e., delivering the one or more non-capturing electrical stimulation pulses during one or more refractory periods with using a test pulse energy levels specified such that it is sufficiently below the most recently determined capture threshold.

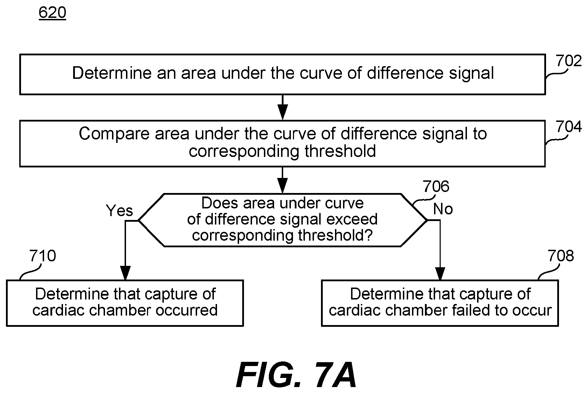

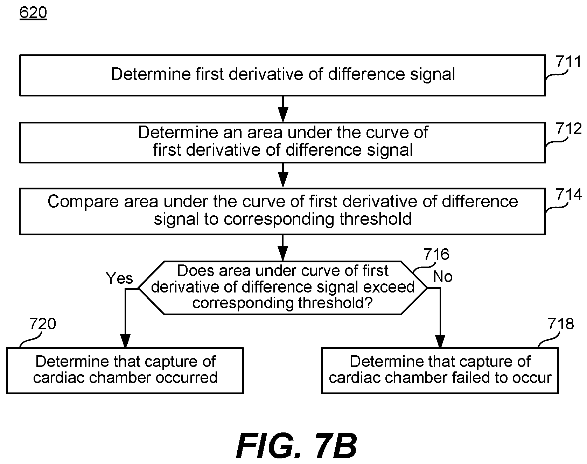

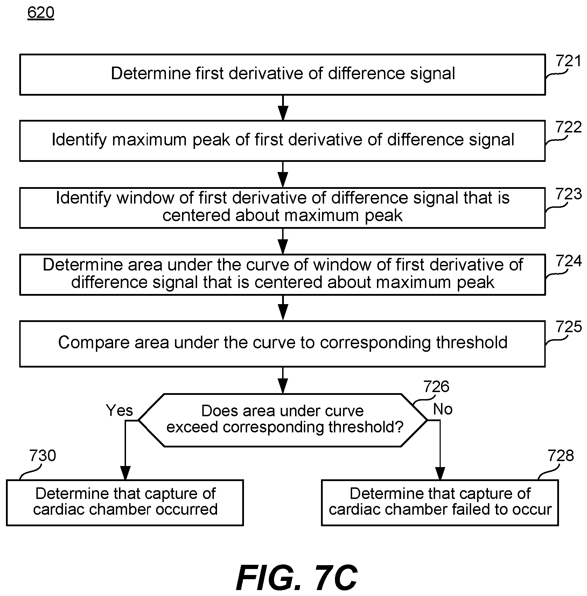

In an embodiment, the determination, at step 620, based on the difference signal (that had been determined at step 618), whether capture of the cardiac chamber occurred or failed to occur responsive to the pacing electrical stimulation pulse(s) having the pacing pulse energy level (that had been delivered at step 614) can involve determining an area under the curve of at least a portion of the difference signal or a first derivative of the difference signal, comparing the area under the curve to a corresponding threshold, and determining that capture of the cardiac chamber occurred when the area under the curve exceeds the corresponding threshold. FIGS. 7A, 7B, and 7C will now be used to describe additional details of step 620 according to various embodiments of the present disclosure. More specifically, these flow diagrams are used describe alternative embodiments for determining, at step 620, based on the difference signal (that had been determined at step 618), whether capture of the cardiac chamber occurred or failed to occur responsive to the pacing electrical stimulation pulse(s) having the pacing pulse energy level (that had been delivered at step 614).

Referring to FIG. 7A, step 702 involves determining an area under the curve of the difference signal, and step 704 involves comparing the area under the curve of the difference signal (that had been determined at step 702) to a corresponding threshold. At step 706 there is a determination of whether the area under the curve of the difference signal (that had been determined at step 704) exceeds the corresponding threshold. While steps 704 and 706 were shown as separate steps, they could have alternatively been shown as a single step. If the answer to the determination at step 706 is No, i.e., if the area under the curve of the difference signal does not exceed the corresponding threshold, then flow goes to step 708 and there is a determination that capture of the cardiac chamber failed to occur. If the answer to the determination at step 706 is Yes, i.e., if the area under the curve of the difference signal exceeds the corresponding threshold, then flow goes to step 710 and there is a determination that capture of the cardiac chamber successfully occurred. Rather than using area under the curve of the difference signal to determine whether or not capture occurred, an area under the curve of a first derivative of the difference signal, or a window thereof, can be used, as described below with reference to FIGS. 7B and 7C.

Referring to FIG. 7B, step 711 involves determining the first derivative of the difference signal, step 712 involves determining an area under the curve of the first derivative of the difference signal, and step 714 involves comparing the area under the curve of the first derivative of the difference signal (that had been determined at step 712) to a corresponding threshold. At step 716 there is a determination of whether the area under the curve of the first derivative of the difference signal (that had been determined at step 714) exceeds a corresponding threshold. While steps 714 and 716 were shown as separate steps, they could have alternatively been shown as a single step. If the answer to the determination at step 716 is No, i.e., if the area under the curve of the first derivative of the difference signal does not exceed the corresponding threshold, then flow goes to step 718 and there is a determination that capture of the cardiac chamber failed to occur. If the answer to the determination at step 716 is Yes, i.e., if the area under the curve of the first derivative of the difference signal exceeds the corresponding threshold, then flow goes to step 720 and there is a determination that capture of the cardiac chamber successfully occurred.

Referring to FIG. 7C, step 721 involves determining a first derivative of the difference signal, step 722 involves identifying a maximum peak of the first derivative of the difference signal, and step 723 involves identifying a window of the first derivative of the difference signal that is centered about the maximum peak. At step 724 an area under the curve (of the window of the first derivative of the difference signal that is centered about the maximum peak) is determined. At step 725 the area under the curve (of the window of the first derivative of the difference signal that is centered about the maximum peak) is compared to a corresponding threshold, and at step 726 there is a determination of whether the area under the curve (of the window of the first derivative of the difference signal that is centered about the maximum peak) exceeds the corresponding threshold.

While steps 725 and 726 were shown as separate steps, they could have alternatively been shown as a single step. If the answer to the determination at step 726 is No, then flow goes to step 728 and there is a determination that capture of the cardiac chamber failed to occur. If the answer to the determination at step 726 is Yes, then flow goes to step 720 and there is a determination that capture of the cardiac chamber successfully occurred.

In certain embodiments described with reference to FIGS. 7A, 7B, 7C, when determining an area under the curve of a signal or a portion thereof (e.g., the difference signal, the first derivative of the difference signal, or a window of the first derivative of the difference signal centered about a maximum peak of the first derivative of the difference signal) positive values of the signal (or portion thereof) are added together without adding any negative values of the signal (or portion thereof). The area under the curve after being determined can be compared to a corresponding threshold to determine whether capture occurred or failed to occur. In still other embodiments, rather than determining an area under the curve and comparing that to a corresponding threshold, a maximum peak of the first derivative of the difference signal is instead compared to a corresponding threshold, without ever determining an area under the curve. More generally, a metric of the difference signal or the first derivative thereof can be compared to a corresponding threshold, wherein the larger the metric the greater the difference between the evoked response morphology of sensed EGM and the polarization artifacts template, and the smaller the metric the lesser the difference between the evoked response morphology of sensed EGM and the polarization artifacts template. In the embodiments described above with reference to FIG. 6, if the metric of the difference signal or the first derivative thereof exceeds the corresponding threshold that that is indicative of capture having occurred, and if the metric of the difference signal or the first derivative thereof does not exceed the corresponding threshold that that is indicative of capture failing to having occurred. Other ways to determine the metric of the difference signal, or the first derivative thereof, include for example, determining an integral of the difference signal, summing measurements (e.g., amplitude measurements) of the signal within one or more specified windows, or adding raw analog-to-digital (ADC) count values corresponding to the signal, but are not limited thereto.

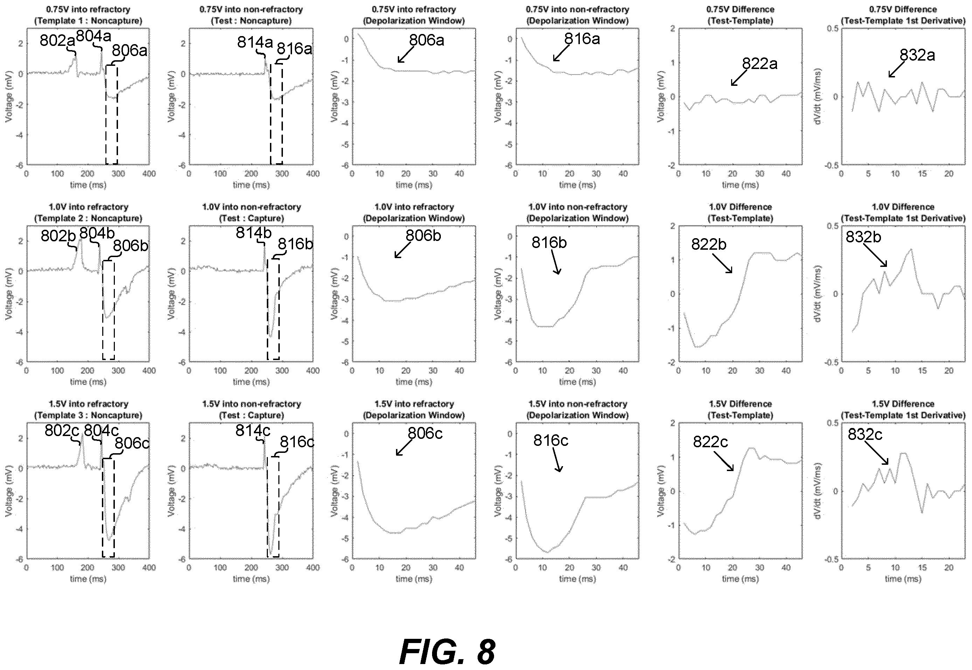

FIG. 8 includes graphs of portions of EGMs that are used to illustrate how embodiments of the present technology, summarized above with reference to the high level flow diagram of FIG. 6, provide for capture management in manners that compensate for polarization artifacts. Initially referring to the graphs in the first row of FIG. 8, the graph in the first column of the first row illustrates how an example polarization artifacts template 806a that can be produced for a first test pulse energy level (i.e., an amplitude of 0.75 V, and a pulse width of 0.40 ms) delivered during an atrial refractory period. More specifically, shown therein is a P-wave 802a corresponding to an atrial depolarization, followed by a pacing artifact 804a resulting from delivery of a non-capturing electrical stimulation pulse having the first test pulse energy level (i.e., an amplitude of 0.75 V, and a pulse width of 0.40 ms) within an atrial refractory period, and followed by polarization artifacts within the window 806a. The polarization artifacts within the window 806a (which can also be referred to as the polarization artifacts window 806a) can be used as a polarization artifacts template corresponding to the first test pulse energy level (i.e., an amplitude of 0.75 V, and a pulse width of 0.40 ms). Accordingly, the polarization artifacts window 806a can also be referred to as the polarization artifacts template 806a corresponding to the first test pulse energy level. The graph in second column of the first row illustrates an evoked response morphology of a sensed EGM following when a pacing electrical stimulation pulse having a first pacing pulse energy level was delivered. More specifically, shown therein is a pacing artifact 814a resulting from delivery of a pacing electrical stimulation pulse having a first pacing pulse energy level (i.e., an amplitude of 0.75 V, and a pulse width of 0.40 ms) that is equal to the first test pulse energy level (i.e., an amplitude of 0.75 V, and a pulse width of 0.40 ms), which delivery was outside an atrial refractory period, followed by an evoked response morphology within the window 816a. The evoked response morphology within the window 816a (which can also be referred to as the evoked response morphology window 816a), in this example, failed to achieve capture of the atrial chamber (i.e., corresponds to non-capture). The graph in the third column of the first row is an enlarged view of the polarization artifacts window 806a (originally shown in the first column of the first row), which is used as the polarization artifacts template corresponding to the first test pulse energy level (i.e., an amplitude of 0.75 V, and a pulse width of 0.40 ms). The graph in the fourth column of the first row is an enlarged view of the evoked response morphology window 816a (originally shown in the second column of the first row). The graph in the fifth column of the first row illustrates a difference signal 822a produced by determining the difference between the evoked response morphology 816a and the polarization artifacts template 806a. The graph in the sixth column (i.e., last column) of the first row illustrates a first derivate signal 832a, which is produced by determining a first derivative of the difference signal 822a (shown in the fifth column of the first row). In accordance with certain embodiments of the present technology, the difference signal 822a, or the first derivative thereof (i.e., the first derivative signal 832a), is used to determine whether capture occurred in response to the pacing electrical stimulation pulse having the first pacing pulse energy level (i.e., an amplitude of 0.75 V, and a pulse width of 0.40 ms), at step 620 of FIG. 6, additional details of which were described above with reference to FIGS. 7A, 7B and 7C. The difference signal 822a (as well as the first derivative signal 832a) is shown as having relatively small inflections with a relative small area under the curve because the evoked response morphology 816a and the polarization artifacts template 806a were very similar to one another, which is indicative of capture failing to be achieved (i.e., is indicative of non-capture).

Reference is now made to the graphs in the second row of FIG. 8. The graph in the first column of the second row illustrates an example polarization artifacts template 806b produced for a second test pulse energy level (i.e., an amplitude of 1.0 V, and a pulse width of 0.40 ms) delivered during an atrial refractory period. More specifically, shown therein is a P-wave 802b corresponding to an atrial depolarization, followed by a pacing artifact 804b resulting from delivery of a non-capturing electrical stimulation pulse having the second test pulse energy level (i.e., an amplitude of 1.0 V, and a pulse width of 0.40 ms) within an atrial refractory period, and followed by polarization artifacts within the window 806b. The polarization artifacts window 806b can be used as a polarization artifacts template corresponding to the second test pulse energy level (i.e., an amplitude of 1.0 V, and a pulse width of 0.40 ms), and thus, can also be referred to as the polarization artifacts template 806b corresponding to the second test pulse energy level. The graph in second column of the second row illustrates a pacing artifact 814b resulting from delivery of a pacing electrical stimulation pulse having a second pacing pulse energy level (i.e., an amplitude of 1.0 V, and a pulse width of 0.40 ms) that is equal to the second test pulse energy level (i.e., an amplitude of 1.0 V, and a pulse width of 0.40 ms), which delivery was outside an atrial refractory period, followed by an evoked response morphology window 816b that successfully achieved capture of the atrial chamber (i.e., corresponds to capture). The graph in the third column of the second row is an enlarged view of the polarization artifacts window 806b (originally shown in the first column of the second row), which is used as the polarization artifacts template corresponding to the second test pulse energy level (i.e., an amplitude of 1.0 V, and a pulse width of 0.40 ms). The graph in the fourth column of the second row is an enlarged view of the evoked response morphology window 816b (originally shown in the second column of the second row). The graph in the fifth column of the second row illustrates a difference signal 822b produced by determining the difference between the evoked response morphology 816b and the polarization artifacts template 806b. The graph in the sixth column (i.e., last column) of the second row illustrates a first derivate signal 832b, which is produced by determining a first derivative of the difference signal 822b (shown in the fifth column of the second row). In accordance with certain embodiments of the present technology, the difference signal 822b, or the first derivative thereof (i.e., the first derivative signal 832b), is used to determine whether capture occurred in response to the pacing electrical stimulation pulse having the second pacing pulse energy level (i.e., an amplitude of 1.0 V, and a pulse width of 0.40 ms), at step 620 of FIG. 6. The difference signal 822b (as well as the first derivative signal 832b) is shown as having relatively large inflections with a relative large area under the curve because the evoked response morphology 816b and the polarization artifacts template 806b were very different from one another, which is indicative of capture successfully being achieved (i.e., is indicative of capture).

Reference is now made to the graphs in the third row of FIG. 8. The graph in the first column of the third row illustrates an example polarization artifacts template 806c produced for a third test pulse energy level (i.e., an amplitude of 1.5 V, and a pulse width of 0.40 ms) delivered during an atrial refractory period. More specifically, shown therein is a P-wave 802c corresponding to an atrial depolarization, followed by a pacing artifact 804c resulting from delivery of a non-capturing electrical stimulation pulse having the third test pulse energy level (i.e., an amplitude of 1.5 V, and a pulse width of 0.40 ms) within an atrial refractory period, and followed a polarization artifacts window 806c, which can be used as and referred to as the polarization artifacts template 806c corresponding to the third test pulse energy level. The graph in second column of the third row illustrates a pacing artifact 814c resulting from delivery of a pacing electrical stimulation pulse having a third pacing pulse energy level (i.e., an amplitude of 1.5 V, and a pulse width of 0.40 ms) that is equal to the third test pulse energy level (i.e., an amplitude of 1.5 V, and a pulse width of 0.40 ms), which delivery was outside an atrial refractory period, followed by an evoked response morphology window 816c that successfully achieved capture of the atrial chamber (i.e., corresponds to capture). The graph in the third column of the third row is an enlarged view of the polarization artifacts window 806c (originally shown in the first column of the third row), which is used as the polarization artifacts template corresponding to the third test pulse energy level (i.e., an amplitude of 1.5 V, and a pulse width of 0.40 ms). The graph in the fourth column of the third row is an enlarged view of the evoked response morphology window 816c (originally shown in the second column of the third row). The graph in the fifth column of the third row illustrates a difference signal 822c produced by determining the difference between the evoked response morphology 816c and the polarization artifacts template 806c. The graph in the sixth column (i.e., last column) of the third row illustrates a first derivate signal 832c, which is produced by determining a first derivative of the difference signal 822c (shown in the fifth column of the third row). In accordance with certain embodiments of the present technology, the difference signal 822c, or the first derivative thereof (i.e., the first derivative signal 832c), is used to determine whether capture occurred in response to the pacing electrical stimulation pulse having the third pacing pulse energy level (i.e., an amplitude of 1.5 V, and a pulse width of 0.40 ms), at step 620 of FIG. 6. The difference signal 822c is shown as having relatively large inflections with a relative large area under the curve because the evoked response morphology 816c and the polarization artifacts template 806c were very different from one another, which is indicative of capture successfully being achieved (i.e., is indicative of capture).

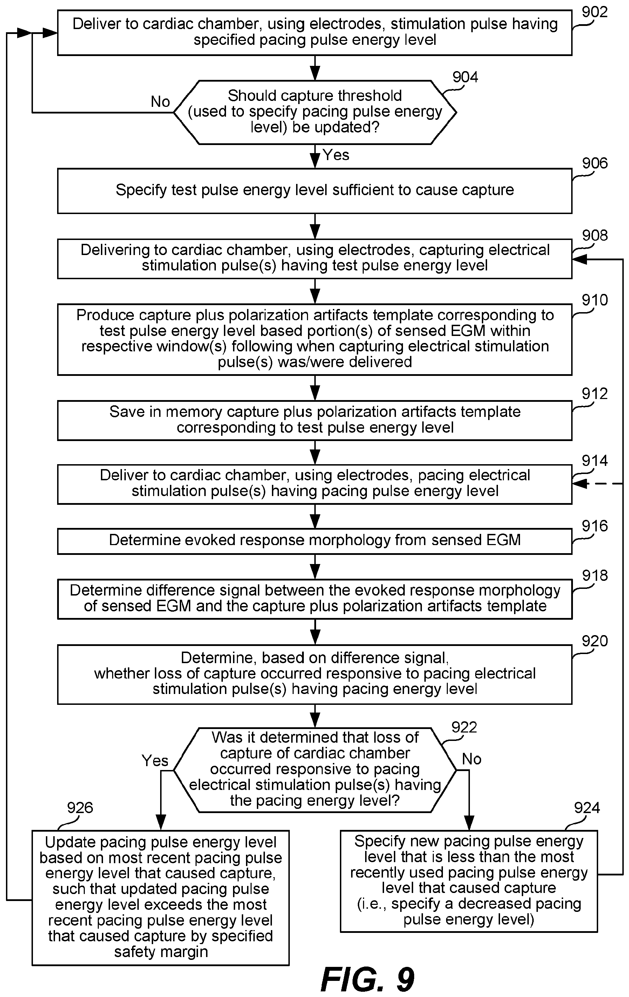

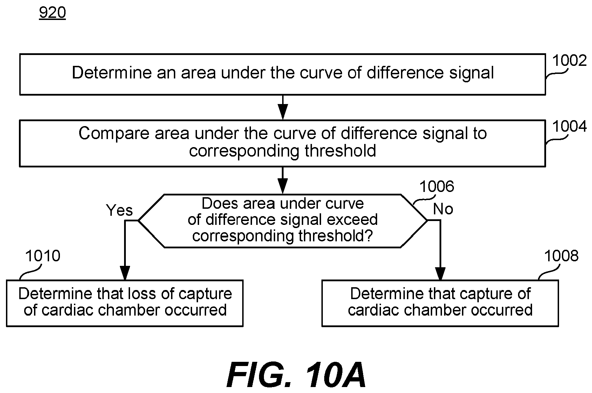

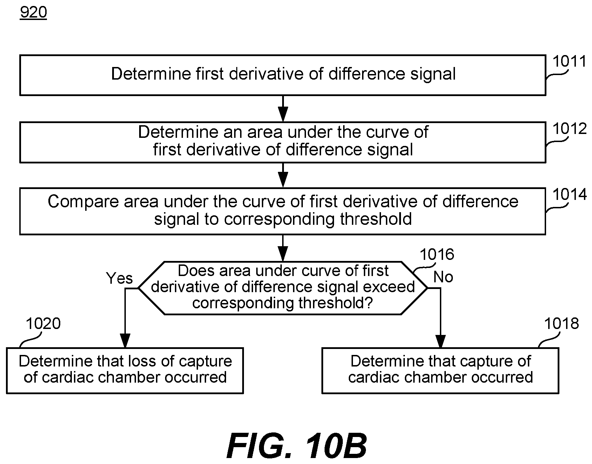

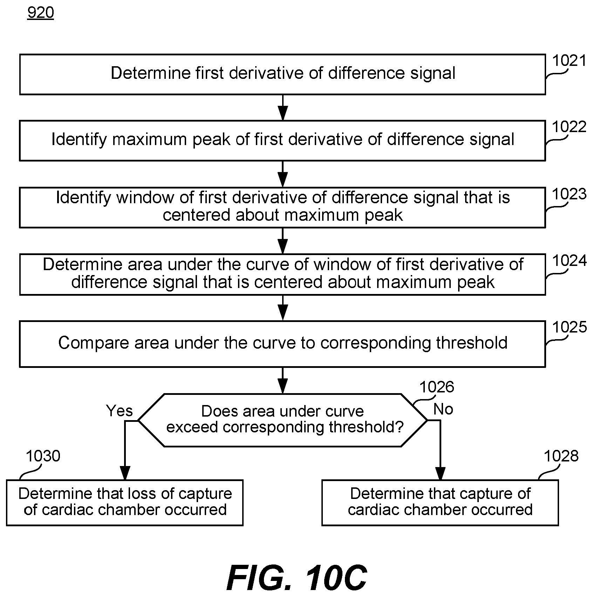

In the above described embodiments, the polarization artifacts template that was determined and saved corresponded to when capture is known to not occur. In alternative embodiments, the polarization artifacts template determined and saved can correspond to when capture is known to occur, e.g., by specifying a test pulse energy level at an instance of step 606 that is well above a known or expected capture threshold, and delivering that high test pulse energy level at an instance of step 608 to produce a corresponding polarization artifacts template at an instance of step 610, which is saved in memory at an instance of step 612. Thereafter, pacing electrical stimulation pulse(s) have a pacing pulse energy (that may be below or above the capture threshold) are delivered at an instance of step 614, and an evoked response morphology is determined at step 616. A difference signal, between the evoked response morphology and the polarization artifacts template, is determined at an instance of step 618, and it used at an instance of step 620 to determine whether capture occurred or failed to occur responsive to the pacing electrical stimulation pulse(s) have the pacing pulse energy. In such embodiments, because the polarization artifacts template corresponds to when capture occurred, the evoked response morphology should be similar to the polarization artifacts template if capture successfully occurred responsive to the pacing electrical stimulation pulse(s) have the pacing pulse energy, and the evoked response morphology should be differ from the polarization artifacts template if capture failed to occur responsive to the pacing electrical stimulation pulse(s) have the pacing pulse energy. Accordingly, in these embodiments, in which there may be stepping down of the pacing pulse energy level during each iteration through a loop in FIG. 6, the area under the curve of the difference signal or the first derivative thereof should be relatively small when capture is achieved, and should be relatively large when capture failed to be achieved, which is the opposite of what is expected when there is a stepping up of the pacing pulse energy level during each iteration through a loop in FIG. 6. Such alternative embodiments are described below in more detail with reference to FIGS. 9 and 10.

Referring to FIG. 9, steps 902 and 904 are the same as steps 602 and 604 discussed above, and thus, need not be described again. At step 906 a test pulse energy level that is sufficient to cause capture is specified, and a pacing pulse energy level that is below the test pulse energy level is specified. While shown as a single step in FIG. 9, step 906 can be shown as two separate steps, one of which involves specifying the test pulse energy level that is sufficient to cause capture, and another of which involves specifying the pacing pulse energy level that is below the test pulse energy level. In certain embodiments, the test pulse energy level that is sufficient to cause capture can be the maximum energy level setting that can be used to deliver cardiac stimulation. In other embodiments, the test pulse energy level that is sufficient to cause capture can be set equal to a previously determined maximum capture threshold plus a specified margin. Other variations are also possible and within the scope of the embodiments described herein. The pacing pulse energy level, that is below the test pulse energy level, can be specified as being a predetermined margin less than the test pulse energy level, or a predetermined percentage of the test pulse energy level, but is not limited thereto. The test pulse energy level sufficient to cause capture, after being specified, is used at step 908 discussed below; and the pacing pulse energy level, after being specified, is used at step 914 discussed below.

Step 908 involves the LP delivering to the cardiac chamber, using its electrodes and the pulse generator, one or more capturing electrical stimulation pulses having the test pulse energy level sufficient to cause capture (that had been specified at step 906), and step 910 involves producing a capture plus polarization artifacts template corresponding to the test pulse energy level based on one or more portions of the sensed EGM within one or more respective windows following when the one or more capturing electrical stimulation pulses was/were delivered at step 908. A reason that stimulation pulses delivered at step 908 should be capturing, i.e., should achieve capture, is so that the template produced at step 910 is representative of both capture and polarization artifacts. In order to ensure that capture is achieved at step 908, the one or more capturing electrical stimulation pulses (having the test pulse energy level) are delivered outside of refractory periods corresponding to one or more windows following one or more depolarizations of the cardiac chamber.

In accordance with certain embodiments, during one cardiac cycle, one capturing electrical stimulation pulse having the test pulse energy level is delivered at step 908, and the capture plus polarization artifacts template corresponding to the test pulse energy level is produced at step 910 based a portion of a sensed EGM within one window (e.g., one 50 msec window) following when the single capturing electrical stimulation pulse was delivered at step 908 (e.g., the 50 msec window can begin 15 msec after the capturing electrical stimulation pulse is delivered). In accordance with other embodiments, during each of a plurality of cardiac cycles, a capturing electrical stimulation pulse having the test pulse energy level is delivered at step 908, and the capture plus polarization artifacts template corresponding to the test pulse energy level is produced at step 910 based portions of a sensed EGM within respective windows (e.g., respective 50 msec windows) following when capturing electrical stimulation pulses were delivered at step 908 (e.g., each of the 50 msec windows can begin 15 msec after each of the capturing electrical stimulation pulses were delivered). For example, at step 910, portions of the sensed EGM, within respective windows following when the capturing electrical stimulation pulses were delivered at step 908, can be averaged (e.g., ensemble averaged) to produce the capture plus polarization artifacts template corresponding to the test pulse energy level. Step 912 involves saving, within a portion of the memory of the LP, the capture plus polarization artifacts template corresponding to the test pulse energy level, which template was determined at step 910. In accordance with certain embodiments, the capture plus polarization artifacts template corresponds to an expected morphology of a sensed EGM responsive to the delivery of one of the capturing electrical stimulation pulses having the test pulse energy level that is above the capture threshold.

Still referring to FIG. 9, step 914 involves the LP delivering to the cardiac chamber (using its electrodes) one or more pacing electrical stimulation pulses having the pacing pulse energy level (that had been specified at step 906), step 916 involves determining an evoked response morphology, and step 918 involves determining a difference signal between the evoked response morphology of the sensed EGM (determined at step 916) and the capture plus polarization artifacts template (produced at step 910).

In accordance with certain embodiments, during one cardiac cycle, one pacing electrical stimulation pulse having the pacing pulse energy level is delivered at step 914, and the evoked response morphology (corresponding to the pacing pulse energy level) is produced at step 916 based a portion of a sensed EGM within one window (e.g., a 50 msec window) following when the single pacing electrical stimulation pulse was delivered at step 914 (e.g., the 50 msec window can begin 15 msec after the single pacing electrical stimulation pulse is delivered). In accordance with other embodiments, during each of a plurality of cardiac cycles, a pacing electrical stimulation pulse having the pacing pulse energy level is delivered at step 914, and the evoked response morphology corresponding to the pacing pulse energy level is produced at step 916 based portions of a sensed EGM within respective windows following when pacing electrical stimulation pulses were delivered at step 914. For example, at step 916, portions of the sensed EGM, within respective windows (e.g., respective 50 msec window) following (e.g., 15 msec after) when the pacing electrical stimulation pulses were delivered at step 914, can be averaged (e.g., ensemble averaged) to determine the evoked response morphology corresponding to the pacing pulse energy level. In accordance with certain embodiments, the evoked response morphology determined at step 916 is temporarily stored in a portion of the memory (e.g., in buffer memory) of the LP before it is used in the subtraction performed at step 918. Depending upon the specific implementation, the aforementioned windows can be longer or shorter that 50 msec, e.g., such windows can have a duration in the range of about 25 msec to 75 msec, but is not limited thereto. Further, such windows need not start at exactly 15 msec following when the pacing electrical stimulation pulse(s) was/were delivered at step 914. For example, such windows can more generally start anywhere within the range of about 2 msec to about 30 msec following when the pacing electrical stimulation pulse(s) was/were delivered at step 914, but is not limited thereto. In other words, in certain embodiments the aforementioned windows can start anywhere within the range of about 2 msec to about 30 msec (e.g., 15 msec) following when the pacing electrical stimulation pulse(s) was/were delivered at step 914, and such windows can have a duration in the range of about 25 msec to 75 msec (e.g., 50 msec), but are not limited thereto.

Still referring to FIG. 9, at step 920 there is a determination, based on the difference signal (that had been determined at step 918), whether loss of capture of the cardiac chamber occurred responsive to the pacing electrical stimulation pulse(s) having the pacing pulse energy level (that had been delivered at step 914). If the difference signal has a morphology that is substantially flat, that means the capture plus polarization artifacts template and the evoked response morphology have substantially the same morphologies as one another, which is indicative pacing electrical stimulation pulse(s) having the pacing pulse energy level (that had been delivered at step 914) successfully achieving capture. On the other hand, if the difference signal has a morphology that is not substantially flat and includes a recognizable and substantial inflection point, that means the capture plus polarization artifacts template and the evoked response morphology have substantially different morphologies than another, which is indicative pacing electrical stimulation pulse(s) having the pacing pulse energy level (that had been delivered at step 914) not achieving capture, and thus, that capture was lost.

At step 922 there is a determination of whether, at step 920, it was determined that loss of capture of the cardiac chamber occurred responsive to the pacing electrical stimulation pulse(s) having the pacing pulse energy level (that had been delivered at step 914). If the answer to the determination at step 922 is No, meaning that pacing electrical stimulation pulse(s) having the pacing pulse energy level achieved capture, then flow goes to step 924. At step 924, a new pacing pulse energy level that is less than the most recently used pacing pulse energy level that caused capture is specified.

After step 924, flow returns to step 908, or alternatively to step 914. Still referring to FIG. 9, when the answer to the determination at step 922 is Yes, flow goes to step 926. At step 926 the pacing pulse energy level (to be used during further pacing of the cardiac chamber) is updated based on the most recently determined capture threshold (which corresponds to the most recent pacing pulse energy level that caused capture). In other words, once there is a determination at step 922 that a loss of capture occurred, then the pacing pulse energy level that most recently (i.e., last) caused the capture to occur (for which the answer to step 922 had been No) is considered to be the new capture threshold. Then, at step 922, the updated pacing pulse energy is determined that exceeds the new capture threshold by the safety margin (aka safety factor). Examples of the safety margin were discussed above with reference to step 626, and thus, needed not be repeated. After the pacing pulse energy level is updated at step 926, flow returns to step 902, at which a stimulation pulse having the specified (and more specifically updated) pacing pulse energy level is delivered to the cardiac chamber by the LP, using electrodes of the LP. The updated pacing pulse energy level continues to be used to pace the cardiac chamber until there is a determination at step 904 that the capture threshold, which had been used to specify the pacing pulse energy level, should again be updated. In the embodiments summarized with reference to FIG. 9, in which there may be stepping down of the pacing pulse energy level during each iteration through a loop in FIG. 9, the area under the curve of the difference signal should be relatively small (and thus, below a corresponding threshold) when capture is achieved, and should be relatively large (and thus, above the corresponding threshold) when capture failed to be achieved (i.e., when there is a loss of capture), which was the opposite of what is expected when there is a stepping up of the pacing pulse energy level during each iteration through a loop in FIG. 6.

In an embodiment, the determination, at step 920, based on the difference signal (that had been determined at step 918), whether capture of the cardiac chamber occurred or failed to occur responsive to the pacing electrical stimulation pulse(s) having the pacing pulse energy level (that had been delivered at step 914) can involve determining an area under the curve of at least a portion of the difference signal or a first derivative of the difference signal, comparing the area under the curve to a corresponding threshold, and determining that loss of capture of the cardiac chamber occurred when the area under the curve exceeds the corresponding threshold.

FIGS. 10A, 10B, and 10C will now be used to describe additional details of step 920 according to various embodiments of the present disclosure. More specifically, these flow diagrams are used describe alternative embodiments for determining, at step 920, based on the difference signal (that had been determined at step 918), whether capture of the cardiac chamber occurred or was lost responsive to the pacing electrical stimulation pulse(s) having the pacing pulse energy level (that had been delivered at step 914).

Referring to FIG. 10A, step 1002 involves determining an area under the curve of the difference signal, and step 1004 involves comparing the area under the curve of the difference signal (that had been determined at step 1002) to a corresponding threshold. At step 1006 there is a determination of whether the area under the curve of the difference signal (that had been determined at step 1004) exceeds the corresponding threshold. While steps 1004 and 1006 were shown as separate steps, they could have alternatively been shown as a single step. If the answer to the determination at step 1006 is No, i.e., if the area under the curve of the difference signal does not exceed corresponding threshold, then flow goes to step 1008 and there is a determination that capture of the cardiac chamber occurred. If the answer to the determination at step 1006 is Yes, i.e., if the area under the curve of the difference signal exceeds the corresponding threshold, then flow goes to step 1010 and there is a determination that loss of capture of the cardiac chamber occurred. Rather than using area under the curve of the difference signal to determine whether or not capture occurred, an area under the curve of a first derivative of the difference signal, or a window thereof, can be used, in a similar manner as was described above with reference to FIGS. 7B and 7C, as explained in more detail below with reference to FIGS. 10B and 10C.

Referring to FIG. 10B, step 1011 involves determining the first derivative of the difference signal, step 1012 involves determining an area under the curve of the first derivative of the difference signal, and step 1014 involves comparing the area under the curve of the first derivative of the difference signal (that had been determined at step 1012) to a corresponding threshold. At step 1016 there is a determination of whether the area under the curve of the first derivative of the difference signal (that had been determined at step 1014) exceeds a corresponding threshold. While steps 1014 and 1016 were shown as separate steps, they could have alternatively been shown as a single step. If the answer to the determination at step 1016 is No, i.e., if the area under the curve of the first derivative of the difference signal does not exceed the corresponding threshold, then flow goes to step 1018 and there is a determination that capture of the cardiac chamber occurred. If the answer to the determination at step 1016 is Yes, i.e., if the area under the curve of the first derivative of the difference signal exceeds the corresponding threshold, then flow goes to step 1020 and there is a determination that loss of capture of the cardiac chamber occurred.

Referring to FIG. 10C, step 1021 involves determining a first derivative of the difference signal, step 1022 involves identifying a maximum peak of the first derivative of the difference signal, and step 1023 involves identifying a window of the first derivative of the difference signal that is centered about the maximum peak. At step 1024 an area under the curve (of the window of the first derivative of the difference signal that is centered about the maximum peak) is determined. At step 1025 the area under the curve (of the window of the first derivative of the difference signal that is centered about the maximum peak) is compared to a corresponding threshold, and at step 1026 there is a determination of whether the area under the curve (of the window of the first derivative of the difference signal that is centered about the maximum peak) exceeds the corresponding threshold.

While steps 1025 and 1026 were shown as separate steps, they could have alternatively been shown as a single step. If the answer to the determination at step 1026 is No, then flow goes to step 1028 and there is a determination that capture of the cardiac chamber occurred. If the answer to the determination at step 1026 is Yes, then flow goes to step 1030 and there is a determination that loss of capture of the cardiac chamber occurred.

In certain embodiments described with reference to FIGS. 10A, 10B, 10C, when determining an area under the curve of a signal or a portion thereof (e.g., the difference signal, the first derivative of the difference signal, or a window of the first derivative of the difference signal centered about a maximum peak of the first derivative of the difference signal) positive values of the signal (or portion thereof) are added together without adding any negative values of the signal (or portion thereof). The area under the curve after being determined can be compared to a corresponding threshold to determine whether capture occurred or failed to occur. In still other embodiments, rather than determining an area under the curve and comparing that to a corresponding threshold, a maximum peak of the first derivative of the difference signal is instead compared to a corresponding threshold, without ever determining an area under the curve. More generally, a metric of the difference signal or the first derivative thereof can be compared to a corresponding threshold, wherein the larger the metric the greater the difference between the evoked response morphology of sensed EGM and the polarization artifacts template, and the smaller the metric the lesser the difference between the evoked response morphology of sensed EGM and the polarization artifacts template. In the embodiments described above with reference to FIG. 9, if the metric of the difference signal or the first derivative thereof exceeds the corresponding threshold that that is indicative of loss of capture having occurred, and if the metric of the difference signal or the first derivative thereof does not exceed the corresponding threshold that that is indicative of capture having occurred. Other ways to determine the metric of the difference signal, or the first derivative thereof, include for example, determining an integral of the difference signal, summing measurements (e.g., amplitude measurements) of the signal within one or more specified windows, or adding raw analog-to-digital (ADC) count values corresponding to the signal, but are not limited thereto.

While many of the embodiments of the present technology described above have been described as being for use with LP type IMDs, embodiments of the present technology, can also be used with other types of IMDs besides an LP. Accordingly, unless specifically limited to use with an LP, the claims should not be limited to use with LP type IMDs.

FIG. 11 shows a block diagram of one embodiment of an IMD (e.g., an LP or ICD) 1101 that is implanted into the patient as part of the implantable cardiac system in accordance with certain embodiments herein. IMD 1101 may be implemented as a full-function biventricular pacemaker, equipped with both atrial and ventricular sensing and pacing circuitry for four chamber sensing and stimulation therapy (including both pacing and shock treatment). Optionally, IMD 1101 may provide full-function cardiac resynchronization therapy. Alternatively, IMD 1101 may be implemented with a reduced set of functions and components. For instance, the IMD may be implemented without ventricular sensing and pacing.

IMD 1101 has a housing 1100 to hold the electronic/computing components. Housing 1100 (which is often referred to as the "can", "case", "encasing", or "case electrode") may be programmably selected to act as the return electrode for certain stimulus modes. Housing 1100 may further include a connector (not shown) with a plurality of terminals 1102, 1104, 1106, 1108, and 1110. The terminals may be connected to electrodes that are located in various locations on housing 1100 or elsewhere within and about the heart. IMD 1101 includes a programmable microcontroller 1120 that controls various operations of IMD 1101, including cardiac monitoring and stimulation therapy. Microcontroller 1120 includes a microprocessor (or equivalent control circuitry), RAM and/or ROM memory, logic and timing circuitry, state machine circuitry, and I/O circuitry.

IMD 1101 further includes a pulse generator 1122 that generates stimulation pulses for delivery by one or more electrodes coupled thereto. Pulse generator 1122 is controlled by microcontroller 1120 via control signal 1124. Pulse generator 1122 may be coupled to the select electrode(s) via an electrode configuration switch 1126, which includes multiple switches for connecting the desired electrodes to the appropriate I/O circuits, thereby facilitating electrode programmability. Switch 1126 is controlled by a control signal 1128 from microcontroller 1120.

In the embodiment of FIG. 11, a single pulse generator 1122 is illustrated. Optionally, the IMD may include multiple pulse generators, similar to pulse generator 1122, where each pulse generator is coupled to one or more electrodes and controlled by microcontroller 1120 to deliver select stimulus pulse(s) to the corresponding one or more electrodes.

Microcontroller 1120 is illustrated as including timing control circuitry 1132 to control the timing of the stimulation pulses (e.g., pacing rate, atrio-ventricular (AV) delay, atrial interconduction (A-A) delay, or ventricular interconduction (V-V) delay, etc.). Timing control circuitry 1132 may also be used for the timing of refractory periods, blanking intervals, noise detection windows, evoked response windows, alert intervals, marker channel timing, and so on. Microcontroller 1120 also has an arrhythmia detector 1134 for detecting arrhythmia conditions and a morphology detector 1136. Although not shown, the microcontroller 1120 may further include other dedicated circuitry and/or firmware/software components that assist in monitoring various conditions of the patient's heart and managing pacing therapies.

IMD 1101 is further equipped with a communication modem (modulator/demodulator) 1140 to enable wireless communication with the remote slave pacing unit. Modem 1140 may include one or more transmitters and two or more receivers as discussed herein in connection with FIG. 2. In one implementation, modem 1140 may use low or high frequency modulation. As one example, modem 1140 may transmit i2i messages and other signals through conductive communication between a pair of electrodes and/or using RF communication. Modem 1140 may be implemented in hardware as part of microcontroller 1120, or as software/firmware instructions programmed into and executed by microcontroller 1120. Alternatively, modem 1140 may reside separately from the microcontroller as a standalone component. In certain embodiments, the modem 1140 includes both a conductive communication transceiver and an RF communication transceiver. While not specifically shown in FIG. 11, such an RF communication transceiver can be coupled to an antenna that enables RF communication signals to be transmitted and received for the purpose of transmitting and receiving messages to and from another IMD.

IMD 1101 includes a sensing circuit 1144 selectively coupled to one or more electrodes, that perform sensing operations, through switch 1126 to detect the presence of cardiac activity in the right chambers of the heart. Sensing circuit 1144 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the cardiac signal of interest. The automatic gain control enables the unit to sense low amplitude signal characteristics of atrial fibrillation. Switch 1126 determines the sensing polarity of the cardiac signal by selectively closing the appropriate switches. In this way, the clinician may program the sensing polarity independent of the stimulation polarity.

The output of sensing circuit 1144 is connected to microcontroller 1120 which, in turn, triggers or inhibits the pulse generator 1122 in response to the presence or absence of cardiac activity. Sensing circuit 1144 receives a control signal 1146 from microcontroller 1120 for purposes of controlling the gain, threshold, polarization charge removal circuitry (not shown), and the timing of any blocking circuitry (not shown) coupled to the inputs of the sensing circuitry.

In the embodiment of FIG. 11, a single sensing circuit 1144 is illustrated. Optionally, the IMD may include multiple sensing circuits, similar to sensing circuit 1144, where each sensing circuit is coupled to one or more electrodes and controlled by microcontroller 1120 to sense electrical activity detected at the corresponding one or more electrodes. Sensing circuit 1144 may operate in a unipolar sensing configuration or in a bipolar sensing configuration.

IMD 1101 further includes an analog-to-digital (A/D) data acquisition system (DAS) 1150 coupled to one or more electrodes via switch 1126 to sample cardiac signals across any pair of desired electrodes. Data acquisition system 1150 is configured to acquire intracardiac electrogram signals, convert the raw analog data into digital data, and store the digital data for later processing and/or telemetric transmission to an external device 1154 (e.g., a programmer, local transceiver, or a diagnostic system analyzer). Data acquisition system 1150 is controlled by a control signal 1156 from the microcontroller 1120.

Microcontroller 1120 is coupled to a memory 1160 by a suitable data/address bus. The programmable operating parameters used by microcontroller 1120 are stored in memory 1160 and used to customize the operation of IMD 1101 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart within each respective tier of therapy.

The operating parameters of IMD 1101 may be non-invasively programmed into memory 1160 through a telemetry circuit 1164 in telemetric communication via communication link 1166 with external device 1154. Telemetry circuit 1164 allows intracardiac electrograms and status information relating to the operation of IMD 1101 (as contained in microcontroller 1120 or memory 1160) to be sent to external device 1154 through communication link 1166. Memory 1160 may also store a table or other data structure that is dynamically updated over time to keep track of the types of i2i communication being used by the IMD 1101 and/or other IMDs with which IMD 1101 communicates. The memory 1160 can also store a capture threshold, various types of templates, such as a polarization artifacts template and/or capture plus polarization artifacts template.

IMD 1101 can optionally include magnet detection circuitry (not shown), coupled to microcontroller 1120, to detect when a magnet is placed over the unit. A magnet may be used by a clinician to perform various test functions of IMD 1101 and/or to signal microcontroller 1120 that external device 1154 is in place to receive or transmit data to microcontroller 1120 through telemetry circuits 1164.

IMD 1101 can optionally include one or more physiological sensors 1170. Such sensors are commonly referred to as "rate-responsive" sensors because they are typically used to adjust pacing stimulation rates according to the exercise state of the patient. However, physiological sensor 1170 may further be used to detect changes in cardiac output, changes in the physiological condition of the heart, or diurnal changes in activity (e.g., detecting sleep and wake states). Signals generated by physiological sensors 1170 are passed to microcontroller 1120 for analysis. Microcontroller 1120 responds by adjusting the various pacing parameters (such as rate, AV Delay, V-V Delay, etc.) at which the atrial and ventricular pacing pulses are administered. While shown as being included within IMD 1101, physiological sensor(s) 1170 may be external to IMD 1101, yet still be implanted within or carried by the patient. Examples of physiologic sensors might include sensors that, for example, sense respiration rate, pH of blood, ventricular gradient, activity, position/posture, minute ventilation (MV), and so forth.

A battery 1172 provides operating power to all of the components in IMD 1101. Battery 1172 is capable of operating at low current drains for long periods of time, and is capable of providing high-current pulses (for capacitor charging) when the patient requires a shock pulse (e.g., in excess of 2 A, at voltages above 2 V, for periods of 10 seconds or more). Battery 1172 also desirably has a predictable discharge characteristic so that elective replacement time can be detected. As one example, IMD 1101 employs lithium/silver vanadium oxide batteries.

IMD 1101 optionally includes an impedance measuring circuit 1174, which can be used for many things, including: lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves; and so forth. Impedance measuring circuit 1174 is coupled to switch 1126 so that any desired electrode may be used.

In this embodiment IMD 1101 optionally includes a shocking circuit 1180 coupled to microcontroller 1120 by a data/address bus 1182.

An aspect of the present technology relates to an LP 102a, 102b, 1101 configured to be implanted in or on a cardiac chamber of a heart, the LP comprising: electrodes 108a, 108b, 1102, 1104, 1106, 1108, 1110; memory 160, 1160; a pulse generator 116, 1112 configured to deliver, using the electrodes, electrical stimulation pulses to the cardiac chamber; a sensing circuit 132, 1144 configured to sense, using the electrodes, an EGM indicative of electrical activity of the heart; and a controller 112, 1120 communicatively coupled to the memory, the pulse generator, and the sensing circuit. In accordance with certain embodiments, the controller 112, 1120 is configured to: cause delivery to the cardiac chamber, using the pulse generator and at least two electrodes of the multiple electrodes, of one or more capturing electrical stimulation pulses having a test pulse energy level that is above a capture threshold; produce a capture plus polarization artifacts template based on one or more portions of the sensed EGM within one or more respective windows following when the one or more capturing electrical stimulation pulses was/were delivered; save, within a portion of the memory, the capture plus polarization artifacts template corresponding to the test pulse energy level; cause delivery to the cardiac chamber, using the pulse generator and at least two electrodes of the multiple electrodes, of one or more pacing electrical stimulation pulses having a pacing pulse energy level that is less than the test pulse energy level; determine a difference signal between an evoked response morphology of the sensed EGM and the capture plus polarization artifacts template, wherein the evoked response morphology of the sensed EGM corresponds to one or more portions of the sensed EGM within one or more respective windows following when the one or more pacing electrical stimulation pulses having the pacing pulse energy level was/were delivered; and determine based on the difference signal whether loss of capture occurred responsive to the one or more pacing electrical stimulation pulses having the pacing energy level that is less than the test pulse energy level.

In accordance with certain embodiments, the controller, in response to determining that loss of capture of the cardiac chamber failed to occur responsive to the one or more pacing electrical stimulation pulses having the pacing pulse energy level that is less than the test pulse energy level, is configured to test a decreased pacing pulse energy level by: causing delivery to the cardiac chamber, using the pulse generator and at least two electrodes of the multiple electrodes, of one or more pacing electrical stimulation pulses having the decreased pacing pulse energy level; determining a further difference signal between a further evoked response morphology of the sensed EGM and the capture plus polarization artifacts template, wherein the further evoked response morphology of the sensed EGM corresponds to one or more portions of the sensed EGM within one or more respective windows following when the one or more pacing electrical stimulation pulses having the decreased pacing pulse energy level was/were delivered; and determining based on the further difference signal whether loss of capture of the cardiac chamber occurred responsive to the one or more pacing electrical stimulation pulses having the decreased pacing energy level.

In accordance with certain embodiments, the controller is configured to: in response to determining that loss of capture of the cardiac chamber failed to occur (i.e., in responding to determining that capture occurred) responsive to the one or more pacing electrical stimulation pulses having the decreased pacing energy level, test one or more further decreased pacing pulse energy levels until loss of capture the cardiac chamber is detected; in response to detecting loss of capture, identify as an updated capture threshold a last decreased pacing energy level that caused capture; and cause delivery to the cardiac chamber, using the pulse generator and at least two of the multiple electrodes, of further pacing electrical stimulation pulses having an updated pacing pulse energy level that exceeds by a safety margin the updated capture threshold.

In accordance with certain embodiments, the controller is configured to save in one or more further portions of the memory the updated capture threshold or the updated pacing pulse energy level, or both.

In accordance with certain embodiments, the controller is configured to: in response to determining that loss of capture of the cardiac chamber failed to occur responsive to the one or more pacing electrical stimulation pulses having the decreased pacing energy level, test one or more further decreased pacing pulse energy levels until loss of capture the cardiac chamber is detected; and in response to detecting loss of capture, cause delivery to the cardiac chamber, using the pulse generator and at least two of the multiple electrodes, of further pacing electrical stimulation pulses having an updated pacing pulse energy level that exceeds by a safety margin a last decreased pacing energy level that caused capture.

In accordance with certain embodiments, the controller is configured to cause delivery of the one or more capturing electrical stimulation pulses outside of one or more refractory periods corresponding to one or more windows following one or more depolarizations of the cardiac chamber.

In accordance with certain embodiments, the controller is configured to determine, based on the difference signal, whether loss of capture of the cardiac chamber occurred, by: determining an area under the curve of the difference signal or of a first derivative of the difference signal; comparing the area under the curve to a corresponding threshold; and determining that loss of capture of the cardiac chamber occurred when the area under the curve exceeds the corresponding threshold.

In accordance with certain embodiments, the controller is configured to determine the area under the curve by adding up positive values of the difference signal or the first derivative of the difference signal.

In accordance with certain embodiments, the controller is configured to determine based on the difference signal whether loss of capture of the cardiac chamber occurred, by: determining a first derivative of the difference signal; identifying a maximum peak of the first derivative of the difference signal; identifying a window of the first derivative of the difference signal that is centered about the maximum peak; determining an area under the curve of the window of the first derivative of the difference signal that is centered about the maximum peak; comparing the area under the curve to a corresponding threshold; and determining that loss of capture of the cardiac chamber occurred when the area under the curve exceeds the corresponding threshold.

In accordance with certain embodiments, the controller is configured to determine, based on the difference signal, whether loss of capture of the cardiac chamber occurred, by: determining a metric of the difference signal or of a first derivative the difference signal, wherein a larger the metric a greater the difference between the evoked response morphology of sensed EGM and the polarization artifacts template, and a smaller the metric a lesser the difference between the evoked response morphology of sensed EGM and the polarization artifacts template; comparing the metric to a corresponding threshold; and determining that loss of capture of the cardiac chamber occurred when the metric exceeds the corresponding threshold.

In accordance with certain embodiments, the LP comprises an atrial LP configured to be implanted in or on an atrial chamber.

Another aspect of the present technology is related to an exemplary method for use by an LP implanted in or on a cardiac chamber of a heart, wherein the LP includes multiple electrodes and memory, and wherein the LP uses at least two of the multiple electrodes for delivering electrical stimulation pulses to the cardiac chamber and at least two of the multiple electrodes for sensing an EGM indicative of electrical activity of the heart, the method comprising: (a) delivering to the cardiac chamber, using at least two of the multiple electrodes, one or more capturing electrical stimulation pulses having a test pulse energy level that is above a capture threshold; (b) producing a capture plus polarization artifacts template based on one or more portions of the sensed EGM within one or more respective windows following when the one or more capturing electrical stimulation pulses was/were delivered; (c) saving, within a portion of the memory, the capture plus polarization artifacts template corresponding to the test pulse energy level; (d) delivering to the cardiac chamber, using at least two of the multiple electrodes, one or more pacing electrical stimulation pulses having a pacing pulse energy level that is less than the test pulse energy level; (e) determining a difference signal between an evoked response morphology of the sensed EGM and the capture plus polarization artifacts template, wherein the evoked response morphology of the sensed EGM corresponds to one or more portions of the sensed EGM within one or more respective windows following when the one or more pacing electrical stimulation pulses having the pacing pulse energy level was/were delivered; and (f) determining based on the difference signal whether loss of capture of the cardiac chamber occurred responsive to the one or more pacing electrical stimulation pulses having the pacing energy level that is less than the test pulse energy level.

In accordance with certain embodiments, the method further comprises: (g) in response to determining that loss of capture of the cardiac chamber failed to occur responsive to the one or more pacing electrical stimulation pulses having the pacing pulse energy level, testing a decreased pacing pulse energy level by repeating each of at least steps (d) through (f), such that the decreased pacing pulse energy level is used at the repeated step (d), and such that the determining at step (f) is whether loss of capture of the cardiac chamber occurred responsive to one or more pacing electrical stimulation pulses having the decreased pacing pulse energy level.

In accordance with certain embodiments, the method further comprises: in response to determining that loss of capture of the cardiac chamber failed to occur responsive to the one or more pacing electrical stimulation pulses having the decreased pacing energy level, testing one or more further decreased pacing pulse energy levels until loss of capture the cardiac chamber is detected; in response to detecting loss of capture, identifying as an updated capture threshold a last decreased pacing energy level that caused capture; and delivering to the cardiac chamber, using two of the multiple electrodes, further pacing electrical stimulation pulses having an updated pacing pulse energy level that exceeds by a safety margin the updated capture threshold.

In accordance with certain embodiments, the method further comprises: saving in one or more further portions of the memory the updated capture threshold or the updated pacing pulse energy level, or both.

In accordance with certain embodiments, the method further comprises: in response to determining that loss of capture of the cardiac chamber failed to occur responsive to the one or more pacing electrical stimulation pulses having the decreased pacing energy level, testing one or more further decreased pacing pulse energy levels until loss of capture the cardiac chamber is detected; and in response to detecting loss of capture, delivering to the cardiac chamber further pacing electrical stimulation pulses having an updated pacing pulse energy level that exceeds by a safety margin a last decreased pacing energy level that caused capture.

In accordance with certain embodiments, the method is periodically performed by the LP in order to update the capture threshold for the LP implanted in or on the cardiac chamber, and wherein the method further comprises: after the capture threshold is updated, updating the pacing pulse energy level based on the capture threshold such that the pacing pulse energy level exceeds the capture threshold by a specified safety margin; and delivering to the cardiac chamber, using the electrodes, further pacing electrical stimulation pulses having the pacing pulse energy level that has been updated.

In accordance with certain embodiments, at step (a) the delivering comprises delivering the one or more capturing electrical stimulation pulses having the test pulse energy level outside of one or more refractory periods corresponding to one or more windows following one or more depolarizations of the cardiac chamber.

In accordance with certain embodiments, at step (f) the determining comprises: determining an area under the curve of the difference signal or of a first derivative of the difference signal; comparing the area under the curve to a corresponding threshold; and determining that loss of capture of the cardiac chamber occurred when the area under the curve of the difference signal exceeds the corresponding threshold.

In accordance with certain embodiments, the determining the area under the curve includes adding up positive values of the difference signal or the first derivative of the difference signal.

In accordance with certain embodiments, at step (f) the determining comprises: determining a first derivative of the difference signal; identifying a maximum peak of the first derivative of the difference signal; identifying a window of the first derivative of the difference signal that is centered about the maximum peak; determining an area under the curve of the window of the first derivative of the difference signal that is centered about the maximum peak; comparing the area under the curve to a corresponding threshold; and determining that loss of capture of the cardiac chamber occurred when the area under the curve exceeds the corresponding threshold.

In accordance with certain embodiments, at step (f) the determining comprises: determining a metric of the difference signal or of a first derivative the difference signal, wherein a larger the metric a greater the difference between the evoked response morphology of sensed EGM and the polarization artifacts template, and a smaller the metric a lesser the difference between the evoked response morphology of sensed EGM and the polarization artifacts template; comparing the metric to a corresponding threshold; and determining that loss of capture of the cardiac chamber occurred when the metric exceeds the corresponding threshold.

In accordance with certain embodiments, the LP that performs the method comprises an atrial LP implanted in or on an atrial chamber.

An aspect of the present technology relates to an LP 102a, 102b, 1101 configured to be implanted in or on a cardiac chamber of a heart, the LP comprising: electrodes 108a, 108b, 1102, 1104, 1106, 1108, 1110; memory 160, 1160; a pulse generator 116, 1112 configured to deliver, using the electrodes, electrical stimulation pulses to the cardiac chamber; a sensing circuit 132, 1144 configured to sense, using the electrodes, an EGM indicative of electrical activity of the heart; and a controller 112, 1120 communicatively coupled to the memory, the pulse generator, and the sensing circuit. In accordance with certain embodiments. In accordance with certain embodiments, the controller is configured to: cause delivery to the cardiac chamber, using the electrodes, of one or more non-capturing electrical stimulation pulses having a test pulse energy level; produce a polarization artifacts template corresponding to the test pulse energy level based on one or more portions of the sensed EGM within one or more respective windows following when the one or more non-capturing electrical stimulation pulses was/were delivered; save, within a portion of the memory, the polarization artifacts template corresponding to the test pulse energy level; cause delivery to the cardiac chamber, using the electrodes, of one or more pacing electrical stimulation pulses having a pacing pulse energy level that is equal to or greater than the test pulse energy level; determine a difference signal between an evoked response morphology of the sensed EGM and the polarization artifacts template, wherein the evoked response morphology of the sensed EGM corresponds to one or more portions of the sensed EGM within one or more respective windows following when the one or more pacing electrical stimulation pulses having the pacing pulse energy level was/were delivered; and determine, based on the difference signal, whether capture of the cardiac chamber occurred or failed to occur responsive to the one or more pacing electrical stimulation pulses having the pacing energy level.

In accordance with certain embodiments, the controller 112, 1120, in response to determining that capture of the cardiac chamber failed to occur responsive to the one or more pacing electrical stimulation pulses having the pacing pulse energy level, is configured to: cause delivery to the cardiac chamber, using the electrodes, of one or more non-capturing electrical stimulation pulses having an increased test pulse energy level; produce a new polarization artifacts template corresponding to the increased test pulse energy level based on one or more portions of the sensed EGM within one or more respective windows following when the one or more non-capturing electrical stimulation pulses having the increased test pulse energy level was/were delivered; save, within the same portion of the memory by overwriting the polarization artifacts template that had previously been saved, the new polarization artifacts template corresponding to the increased test pulse energy level; cause delivery to the cardiac chamber, using the electrodes, of one or more pacing electrical stimulation pulses having an increased pacing pulse energy level that is equal to or greater than the increased test pulse energy level; determine a difference signal between an evoked response morphology of the sensed EGM and the new polarization artifacts template, wherein the evoked response morphology of the sensed EGM corresponds to one or more portions of the sensed EGM within one or more respective windows following when the one or more pacing electrical stimulation pulses having the increased pacing pulse energy level was/were delivered; and determine, based on the difference signal, whether capture of the cardiac chamber occurred or failed to occur responsive to the one or more pacing electrical stimulation pulses having the increased pacing energy level.

In accordance with certain embodiments, the controller 112, 1120 is configured to cause delivery of the one or more non-capturing electrical stimulation pulses during one or more refractory periods corresponding to one or more windows following one or more depolarizations of the cardiac chamber.

In accordance with certain embodiments, the controller 112, 1120 is configured to periodically update a capture threshold for the LP, and after the capture threshold is updated, is configured to: update the pacing pulse energy level based on the capture threshold such that the pacing pulse energy level exceeds the capture threshold by a specified safety margin; and cause delivery to the cardiac chamber, using the electrodes, of further pacing electrical stimulation pulses having the pacing pulse energy level that has been updated.

In accordance with certain embodiments, the controller 112, 1120 is configured to determine, based on the difference signal, whether capture of the cardiac chamber occurred or failed to occur, by: determining an area under the curve of the difference signal; comparing the area under the curve of the difference signal to a corresponding threshold; and determining that capture of the cardiac chamber occurred when the area under the curve of the difference signal exceeds the corresponding threshold.

In accordance with certain embodiments, the controller 112, 1120 is configured to determine, based on the difference signal, whether capture of the cardiac chamber occurred or failed to occur, by: determining a first derivative of the difference signal; determining an area under the curve of the first derivative of the difference signal; comparing the area under the curve of the first derivative of the difference signal to a corresponding threshold; and determining that capture of the cardiac chamber occurred when the area under the curve of the first derivative of the difference signal exceeds the corresponding threshold.

In accordance with certain embodiments, the controller 112, 1120 is configured to determine, based on the difference signal, whether capture of the cardiac chamber occurred or failed to occur, by: determining a first derivative of the difference signal; identifying a maximum peak of the first derivative of the difference signal; identifying a window of the first derivative of the difference signal that is centered about the maximum peak; and determining an area under the curve of the window of the first derivative of the difference signal that is centered about the maximum peak; and comparing the area under the curve to a corresponding threshold; and determining that capture of the cardiac chamber occurred when the area under the curve exceeds the corresponding threshold.

In accordance with certain embodiments, the LP comprises an atrial LP configured to be implanted in or on an atrial chamber.

In accordance with certain embodiments, whenever a difference signal is determined, between the evoked response morphology of the sensed EGM and the polarization artifacts template, the pacing pulse energy level of the one or more pacing electrical stimulation pulses delivered and used to sense the evoked response morphology, is equal to the test pulse energy level of the non-capturing electrical stimulation pulses delivered and used to produce the polarization artifacts template.

In accordance with certain embodiments, whenever a difference signal is determined, between the evoked response morphology of the sensed EGM and the polarization artifacts template, the pacing pulse energy level of the one or more pacing electrical stimulation pulses delivered and used to sense the evoked response morphology, is greater than the test pulse energy level of the non-capturing electrical stimulation pulses delivered and used to produce the polarization artifacts template.

Another aspect of the present technology relates to an exemplary method for use by an LP 102a, 102b, 1101 implanted in or on a cardiac chamber of a heart, wherein the LP includes electrodes 108a, 108b, 1102, 1104, 1106, 1108, 1110 and memory 160, 1160, and wherein the LP uses the same electrodes for delivering electrical stimulation pulses to the cardiac chamber and for sensing an EGM indicative of electrical activity of the heart. In accordance with certain embodiments, the method comprises: (a) delivering to the cardiac chamber, using the electrodes, one or more non-capturing electrical stimulation pulses having a test pulse energy level; (b) producing a polarization artifacts template corresponding to the test pulse energy level based on one or more portions of the sensed EGM within one or more respective windows following when the one or more non-capturing electrical stimulation pulses was/were delivered; (c) saving, within a portion of the memory, the polarization artifacts template corresponding to the test pulse energy level; (d) delivering to the cardiac chamber, using the electrodes, one or more pacing electrical stimulation pulses having a pacing pulse energy level that is equal to or greater than the test pulse energy level; (e) determining a difference signal between an evoked response window morphology of the sensed EGM and the polarization artifacts template, wherein the evoked response morphology of the sensed EGM corresponds to one or more portions of the sensed EGM within one or more respective windows following when the one or more pacing electrical stimulation pulses having the pacing pulse energy level was/were delivered; and (f) determining, based on the difference signal, whether capture of the cardiac chamber occurred or failed to occur responsive to the one or more pacing electrical stimulation pulses having the pacing energy level.

In accordance with certain embodiments, the method further comprises: (g) in response to determining that capture of the cardiac chamber failed to occur responsive to the one or more pacing electrical stimulation pulses having the pacing pulse energy level, repeating each of steps (a) through (f), such that an increased test pulse energy level is used at the repeated step (a), such that a new polarization artifacts template corresponding to the increased test pulse energy level is produced at the repeated step (b) and is saved at the repeated step (c) in the same portion of the memory by overwriting the polarization artifacts template that had been saved in a preceding instance of step (c), such that an increased pacing pulse energy level is used at the repeated step (d), and such that the determining at step (f) is whether capture of the cardiac chamber occurred or failed to occur responsive to one or more pacing electrical stimulation pulses having the increased pacing pulse energy level.

In accordance with certain embodiments, at step (a) the delivering comprises delivering the one or more non-capturing electrical stimulation pulses having the test pulse energy level during one or more refractory periods corresponding to one or more windows following one or more depolarizations of the cardiac chamber.

In accordance with certain embodiments, the method is periodically performed by the LP in order to update a capture threshold for the LP implanted in or on the cardiac chamber, and wherein the method further comprises: after the capture threshold is updated, updating the pacing pulse energy level based on the capture threshold such that the pacing pulse energy level exceeds the capture threshold by a specified safety margin; and delivering to the cardiac chamber, using the electrodes, further pacing electrical stimulation pulses having the pacing pulse energy level that has been updated.

In accordance with certain embodiments, at step (f) the determining comprises: determining an area under the curve of the difference signal; comparing the area under the curve of the difference signal to a corresponding threshold; and determining that capture of the cardiac chamber occurred when the area under the curve of the difference signal exceeds the corresponding threshold.

In accordance with certain embodiments, at step (f) the determining comprises: determining a first derivative of the difference signal; determining an area under the curve of the first derivative of the difference signal; comparing the area under the curve of the first derivative of the difference signal to a corresponding threshold; and determining that capture of the cardiac chamber occurred when the area under the curve of the first derivative of the difference signal exceeds the corresponding threshold.

In accordance with certain embodiments, at step (f) the determining comprises: determining a first derivative of the difference signal; identifying a maximum peak of the first derivative of the difference signal; identifying a window of the first derivative of the difference signal that is centered about the maximum peak; and determining an area under the curve of the window of the first derivative of the difference signal that is centered about the maximum peak; and comparing the area under the curve to a corresponding threshold; and determining that capture of the cardiac chamber occurred when the area under the curve exceeds the corresponding threshold.

In accordance with certain embodiments, the LP that performs the method comprises an atrial LP implanted in or on an atrial chamber.

In accordance with certain embodiments, whenever a difference signal is determined, between the evoked response morphology of the sensed EGM and the polarization artifacts template, the pacing pulse energy level of the one or more pacing electrical stimulation pulses delivered and used to sense the evoked response morphology, is equal to the test pulse energy level of the non-capturing electrical stimulation pulses delivered and used to produce the polarization artifacts template.

In accordance with certain embodiments, whenever a difference signal is determined, between the evoked response morphology of the sensed EGM and the polarization artifacts template, the pacing pulse energy level of the one or more pacing electrical stimulation pulses delivered and used to sense the evoked response morphology, is greater than the test pulse energy level of the non-capturing electrical stimulation pulses delivered and used to produce the polarization artifacts template.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

It is to be understood that the above description is intended to be illustrative, and not restrictive. scope of the present invention should, therefore, be determined with reference to the appended claims.

## Claims

1. A leadless pacemaker, LP, (102a, 102b, 1101) configured to be implanted in or on a cardiac chamber of a heart, the LP (102a, 102b, 1101) comprising:
multiple electrodes (108a, 108b, 1100, 1102, 1104, 1106, 1108, 1110);
a memory (160, 1160);
a pulse generator (116, 1122) configured to deliver, using at least two electrodes of the multiple electrodes (108a, 108b, 1100, 1102, 1104, 1106, 1108, 1110), electrical stimulation pulses to the cardiac chamber;
a sensing circuit (132, 1144) configured to sense, using at least two electrodes of the multiple electrodes (108a, 108b, 1100, 1102, 1104, 1106, 1108, 1110), an electrogram, EGM, indicative of electrical activity of the heart; and
a controller (112, 1120) communicatively coupled to the memory (160, 1160), the pulse generator (116, 1122), and the sensing circuit (132, 1144), the controller (112, 1120) configured to:
cause delivery to the cardiac chamber, using the pulse generator (116, 1122) and at least two electrodes of the multiple electrodes (108a, 108b, 1100, 1102, 1104, 1106, 1108, 1110), of one or more capturing electrical stimulation pulses having a test pulse energy level that is above a capture threshold;
produce a capture plus polarization artifacts template based on one or more portions of the sensed EGM within one or more respective windows following when the one or more capturing electrical stimulation pulses was/were delivered;
save, within a portion of the memory (160, 1160), the capture plus polarization artifacts template corresponding to the test pulse energy level;
cause delivery to the cardiac chamber, using the pulse generator (116, 1122) and at least two electrodes of the multiple electrodes (108a, 108b, 1100, 1102, 1104, 1106, 1108, 1110), of one or more pacing electrical stimulation pulses having a pacing pulse energy level that is less than the test pulse energy level;
determine a difference signal between an evoked response morphology of the sensed EGM and the capture plus polarization artifacts template, wherein the evoked response morphology of the sensed EGM corresponds to one or more portions of the sensed EGM within one or more respective windows following when the one or more pacing electrical stimulation pulses having the pacing pulse energy level was/were delivered; and
determine based on the difference signal whether loss of capture occurred responsive to the one or more pacing electrical stimulation pulses having the pacing energy level that is less than the test pulse energy level.

2. The LP (102a, 102b, 1101) of claim 1, wherein the controller (112, 1120), in response to determining that loss of capture of the cardiac chamber failed to occur responsive to the one or more pacing electrical stimulation pulses having the pacing pulse energy level that is less than the test pulse energy level, is configured to test a decreased pacing pulse energy level by:
causing delivery to the cardiac chamber, using the pulse generator (116, 1122) and at least two electrodes of the multiple electrodes (108a, 108b, 1100, 1102, 1104, 1106, 1108, 1110), of one or more pacing electrical stimulation pulses having the decreased pacing pulse energy level;
determining a further difference signal between a further evoked response morphology of the sensed EGM and the capture plus polarization artifacts template, wherein the further evoked response morphology of the sensed EGM corresponds to one or more portions of the sensed EGM within one or more respective windows following when the one or more pacing electrical stimulation pulses having the decreased pacing pulse energy level was/were delivered; and
determining based on the further difference signal whether loss of capture of the cardiac chamber occurred responsive to the one or more pacing electrical stimulation pulses having the decreased pacing energy level.

3. The LP (102a, 102b, 1101) of any one of claims 1 or 2, wherein the controller (112, 1120) is configured to:
in response to determining that loss of capture of the cardiac chamber failed to occur responsive to the one or more pacing electrical stimulation pulses having the decreased pacing energy level, test one or more further decreased pacing pulse energy levels until loss of capture the cardiac chamber is detected;
in response to detecting loss of capture, identify as an updated capture threshold a last decreased pacing energy level that caused capture; and
cause delivery to the cardiac chamber, using the pulse generator (116, 1122) and at least two of the multiple electrodes (108a, 108b, 1100, 1102, 1104, 1106, 1108, 1110), of further pacing electrical stimulation pulses having an updated pacing pulse energy level that exceeds by a safety margin the updated capture threshold.

4. The LP (102a, 102b, 1101) of claim 3, wherein the controller (112, 1120) is configured to save in one or more further portions of the memory the updated capture threshold or the updated pacing pulse energy level, or both.

5. The LP (102a, 102b, 1101) of claim 2, wherein the controller (112, 1120) is configured to:
in response to determining that loss of capture of the cardiac chamber failed to occur responsive to the one or more pacing electrical stimulation pulses having the decreased pacing energy level, test one or more further decreased pacing pulse energy levels until loss of capture the cardiac chamber is detected; and
in response to detecting loss of capture, cause delivery to the cardiac chamber, using the pulse generator (116, 1122) and at least two of the multiple electrodes (108a, 108b, 1100, 1102, 1104, 1106, 1108, 1110), of further pacing electrical stimulation pulses having an updated pacing pulse energy level that exceeds by a safety margin a last decreased pacing energy level that caused capture.

6. The LP (102a, 102b, 1101) of any one of claims 1 through 5, wherein the controller (112, 1120) is configured to cause delivery of the one or more capturing electrical stimulation pulses outside of one or more refractory periods corresponding to one or more windows following one or more depolarizations of the cardiac chamber.

7. The LP (102a, 102b, 1101) of any one of claims 1 through 6, wherein the controller (112, 1120) is configured to determine, based on the difference signal, whether loss of capture of the cardiac chamber occurred, by:
determining an area under the curve of the difference signal or of a first derivative of the difference signal;
comparing the area under the curve to a corresponding threshold; and
determining that loss of capture of the cardiac chamber occurred when the area under the curve exceeds the corresponding threshold.

8. The LP (102a, 102b, 1101) of any one of claims 1 through 6, wherein the controller (112, 1120) is configured to determine based on the difference signal whether loss of capture of the cardiac chamber occurred, by:
determining a first derivative of the difference signal;
identifying a maximum peak of the first derivative of the difference signal;
identifying a window of the first derivative of the difference signal that is centered about the maximum peak;
determining an area under the curve of the window of the first derivative of the difference signal that is centered about the maximum peak;
comparing the area under the curve to a corresponding threshold; and
determining that loss of capture of the cardiac chamber occurred when the area under the curve exceeds the corresponding threshold.

9. The LP (102a, 102b, 1101) of any one of claims 1 through 6, wherein the controller (112, 1120) is configured to determine, based on the difference signal, whether loss of capture of the cardiac chamber occurred, by:
determining a metric of the difference signal or of a first derivative the difference signal, wherein a larger the metric a greater the difference between the evoked response morphology of sensed EGM and the polarization artifacts template, and a smaller the metric a lesser the difference between the evoked response morphology of sensed EGM and the polarization artifacts template;
comparing the metric to a corresponding threshold; and
determining that loss of capture of the cardiac chamber occurred when the metric exceeds the corresponding threshold.

10. The LP (102a, 102b, 1101) of any one of claims 1 through 9, wherein the LP (102a, 102b, 1101) comprises an atrial LP (102a) configured to be implanted in or on an atrial chamber.

11. The LP (102a, 102b, 1101) of any one of claims 1 through 9, wherein the LP (102a, 102b, 1101) comprises a ventricular LP (102b) configured to be implanted in or on an atrial chamber.

12. The LP (102a, 102b, 1101) of any one of claims 1 through 9, wherein the controller (112, 1120) is configured to produce the capture plus polarization artifacts template by averaging portions of the sensed EGM within respective windows following when each of a plurality of the capturing electrical stimulation pulses are delivered.

13. The LP (102a, 102b, 1101) of any one of claims 1 through 12, wherein the multiple electrodes (108a, 108b, 1100, 1102, 1104, 1106, 1108, 1110) comprise only two electrodes (108a, 108b) that are used by the pulse generator (116, 1122) to deliver the electrical stimulation pulses to the cardiac chamber and are also used by the sensing circuit (132, 1144) to sense the EGM indicative of electrical activity of the heart.

14. The LP (102a, 102b, 1101) of claim 13, wherein the only two electrodes (108a, 108b) comprise a tip electrode (108a) and a ring electrode (108b).

15. The LP (102a, 102b, 1101) of any one of claims 1 through 12, wherein:
the multiple electrodes (108a, 108b, 1100, 1102, 1104, 1106, 1108, 1110) comprise three or more electrodes;
a first set of the three or more electrodes are used by the pulse generator (116, 1122) to deliver the electrical stimulation pulses to the cardiac chamber; and
a second set of the three or more electrodes that differs from the first set is used by the sensing circuit (132, 1144) to sense the EGM indicative of electrical activity of the heart.

## Patentansprüche

1. Leitungsloser Schrittmacher, LP, (102a, 102b, 1101), der dazu konfiguriert ist, in oder an einer Herzkammer eines Herzens implantiert zu sein, wobei der LP (102a, 102b, 1101) Folgendes umfasst:
mehrere Elektroden (108a, 108b, 1100, 1102, 1104, 1106, 1108, 1110);
einen Speicher (160, 1160);
einen Impulsgenerator (116, 1122), der dazu konfiguriert ist, unter Verwendung mindestens zweier Elektroden der mehreren Elektroden (108a, 108b, 1100, 1102, 1104, 1106, 1108, 1110) elektrische Stimulationsimpulse an die Herzkammer abzugeben; eine Erfassungsschaltung (132, 1144), die dazu konfiguriert ist, unter Verwendung mindestens zweier Elektroden der mehreren Elektroden (108a, 108b, 1100, 1102, 1104, 1106, 1108, 1110) ein Elektrogramm, EGM, zu erfassen, das eine elektrische Aktivität des Herzens angibt; und
eine Steuerung (112, 1120), die kommunikativ an den Speicher (160, 1160), den Impulsgenerator (116, 1122) und die Erfassungsschaltung (132, 1144) gekoppelt ist, wobei die Steuerung (112, 1120) zu Folgendem konfiguriert ist:
Veranlassen einer Abgabe an die Herzkammer, unter Verwendung des Impulsgenerators (116, 1122) und mindestens zweier Elektroden der mehreren Elektroden (108a, 108b, 1100, 1102, 1104, 1106, 1108, 1110), eines oder mehrerer aufnehmender elektrischer Stimulationsimpulse, die ein Prüfimpulsenergieniveau aufweisen, das über einem Aufnahmeschwellenwert liegt;
Produzieren einer Aufnahme-plus-Polarisationsartefaktmatrize basierend auf einem oder mehreren Abschnitten des erfassten EGM innerhalb eines oder mehrerer jeweiliger Fenster, die folgen, wenn der eine oder die mehreren aufnehmenden elektrischen Stimulationsimpulse abgegeben wurde/wurden;
Speichern, innerhalb eines Abschnitts des Speichers (160, 1160), der Aufnahme-plus-Polarisationsartefaktmatrize, die dem Prüfimpulsenergieniveau entspricht;
Veranlassen einer Abgabe an die Herzkammer, unter Verwendung des Impulsgenerators (116, 1122) und mindestens zweier Elektroden der mehreren Elektroden (108a, 108b, 1100, 1102, 1104, 1106, 1108, 1110), eines oder mehrerer schrittmachender elektrischer Stimulationsimpulse, die ein schrittmachendes Impulsenergieniveau aufweisen, das geringer als das Prüfimpulsenergieniveau ist;
Bestimmen eines Differenzsignals zwischen einer evozierten Reaktionsmorphologie des erfassten EGM und der Aufnahme-plus-Polarisationsartefaktmatrize, wobei die evozierte Reaktionsmorphologie des erfassten EGM einem oder mehreren Abschnitten des erfassten EGM innerhalb eines oder mehrerer jeweiliger Fenster entspricht, die folgen, wenn der eine oder die mehreren schrittmachenden elektrischen Stimulationsimpulse, die das schrittmachende Impulsenergieniveau aufweisen, abgegeben wurde/wurden; und
Bestimmen, basierend auf dem Differenzsignal, ob ein Aufnahmeverlust als Reaktion auf den einen oder die mehreren schrittmachenden elektrischen Stimulationsimpulse, die das schrittmachende Energieniveau aufweisen, das geringer als das Prüfimpulsenergieniveau ist, aufgetreten ist.

2. LP (102a, 102b, 1101) nach Anspruch 1, wobei die Steuerung (112, 1120) als Reaktion auf Bestimmen, dass ein Aufnahmeverlust der Herzkammer als Reaktion auf den einen oder die mehreren schrittmachenden elektrischen Stimulationsimpulse, die das schrittmachende Impulsenergieniveau aufweisen, das geringer als das Prüfimpulsenergieniveau ist, nicht aufgetreten ist, dazu konfiguriert ist, ein verringertes schrittmachendes Impulsenergieniveau durch Folgendes zu prüfen:
Veranlassen einer Abgabe an die Herzkammer, unter Verwendung des Impulsgenerators (116, 1122) und mindestens zweier Elektroden der mehreren Elektroden (108a, 108b, 1100, 1102, 1104, 1106, 1108, 1110), eines oder mehrerer schrittmachender elektrischer Stimulationsimpulse, die das verringerte schrittmachende Impulsenergieniveau aufweisen;
Bestimmen eines weiteren Differenzsignals zwischen einer weiteren evozierten Reaktionsmorphologie des erfassten EGM und der Aufnahme-plus-Polarisationsartefaktmatrize, wobei die weitere evozierte Reaktionsmorphologie des erfassten EGM einem oder mehreren Abschnitten des erfassten EGM innerhalb eines oder mehrerer jeweiliger Fenster entspricht, die folgen, wenn der eine oder die mehreren schrittmachenden elektrischen Stimulationsimpulse, die das verringerte schrittmachende Impulsenergieniveau aufweisen, abgegeben wurde/wurden; und
Bestimmen, basierend auf dem weiteren Differenzsignal, ob ein Aufnahmeverlust der Herzkammer als Reaktion auf den einen oder die mehreren schrittmachenden elektrischen Stimulationsimpulse, die das verringerte schrittmachende Energieniveau aufweisen, aufgetreten ist.

3. LP (102a, 102b, 1101) nach einem der Ansprüche 1 oder 2, wobei die Steuerung (112, 1120) zu Folgendem konfiguriert ist:
als Reaktion auf Bestimmen, dass ein Aufnahmeverlust der Herzkammer als Reaktion auf den einen oder die mehreren schrittmachenden elektrischen Stimulationsimpulse, die das verringerte schrittmachende Energieniveau aufweisen, nicht aufgetreten ist, Prüfen eines oder mehrerer weiterer verringerter schrittmachender Impulsenergieniveaus, bis der Aufnahmeverlust der Herzkammer erkannt wird;
als Reaktion auf das Erkennen des Aufnahmeverlusts, Identifizieren, als einen aktualisierten Aufnahmeschwellenwert, eines letzten verringerten schrittmachenden Energieniveaus, das die Aufnahme verursacht hat; und
Veranlassen einer Abgabe an die Herzkammer, unter Verwendung des Impulsgenerators (116, 1122) und mindestens zweier der mehreren Elektroden (108a, 108b, 1100, 1102, 1104, 1106, 1108, 1110), weiterer schrittmachender elektrischer Stimulationsimpulse, die ein aktualisiertes schrittmachendes Impulsenergieniveau aufweisen, das den aktualisierten Aufnahmeschwellenwert um eine Sicherheitstoleranz überschreitet.

4. LP (102a, 102b, 1101) nach Anspruch 3, wobei die Steuerung (112, 1120) dazu konfiguriert ist, in einem oder mehreren weiteren Abschnitten des Speichers den aktualisierten Aufnahmeschwellenwert oder das aktualisierte schrittmachende Impulsenergieniveau oder beide zu speichern.

5. LP (102a, 102b, 1101) nach Anspruch 2, wobei die Steuerung (112, 1120) zu Folgendem konfiguriert ist:
als Reaktion auf Bestimmen, dass ein Aufnahmeverlust der Herzkammer als Reaktion auf den einen oder die mehreren schrittmachenden elektrischen Stimulationsimpulse, die das verringerte schrittmachende Energieniveau aufweisen, nicht aufgetreten ist, Prüfen eines oder mehrerer weiterer verringerter schrittmachender Impulsenergieniveaus, bis der Aufnahmeverlust die Herzkammer erkannt wird; und
als Reaktion auf das Erkennen des Aufnahmeverlusts, Veranlassen einer Abgabe an die Herzkammer, unter Verwendung des Impulsgenerators (116, 1122) und mindestens zweier der mehreren Elektroden (108a, 108b, 1100, 1102, 1104, 1106, 1108, 1110), weiterer schrittmachender elektrischer Stimulationsimpulse, die ein aktualisiertes schrittmachendes Impulsenergieniveau aufweisen, das ein letztes verringertes schrittmachendes Energieniveau, das die Aufnahme veranlasst hat, um eine Sicherheitstoleranz überschreitet.

6. LP (102a, 102b, 1101) nach einem der Ansprüche 1 bis einschließlich 5, wobei die Steuerung (112, 1120) dazu konfiguriert ist, eine Abgabe des einen oder der mehreren aufnehmenden elektrischen Stimulationsimpulse außerhalb einer oder mehrerer Refraktärperioden zu veranlassen, die einem oder mehreren Fenstern entsprechen, die auf eine oder mehrere Depolarisierungen der Herzkammer folgen.

7. LP (102a, 102b, 1101) nach einem der Ansprüche 1 bis einschließlich 6, wobei die Steuerung (112, 1120) dazu konfiguriert ist, basierend auf dem Differenzsignal durch Folgendes zu bestimmen, ob der Aufnahmeverlust der Herzkammer aufgetreten ist:
Bestimmen einer Fläche unter der Kurve des Differenzsignals oder einer ersten Ableitung des Differenzsignals;
Vergleichen der Fläche unter der Kurve mit einem entsprechenden Schwellenwert; und
Bestimmen, dass der Aufnahmeverlust der Herzkammer aufgetreten ist, wenn die Fläche unter der Kurve den entsprechenden Schwellenwert überschreitet.

8. LP (102a, 102b, 1101) nach einem der Ansprüche 1 bis einschließlich 6, wobei die Steuerung (112, 1120) dazu konfiguriert ist, basierend auf dem Differenzsignal durch Folgendes zu bestimmen, ob der Aufnahmeverlust der Herzkammer aufgetreten ist:
Bestimmen einer ersten Ableitung des Differenzsignals;
Identifizieren einer maximalen Spitze der ersten Ableitung des Differenzsignals;
Identifizieren eines Fensters der ersten Ableitung des Differenzsignals, das um die maximale Spitze zentriert ist;
Bestimmen einer Fläche unter der Kurve des Fensters der ersten Ableitung des Differenzsignals, die um die maximale Spitze zentriert ist;
Vergleichen der Fläche unter der Kurve mit einem entsprechenden Schwellenwert; und
Bestimmen, dass der Aufnahmeverlust der Herzkammer aufgetreten ist, wenn die Fläche unter der Kurve den entsprechenden Schwellenwert überschreitet.

9. LP (102a, 102b, 1101) nach einem der Ansprüche 1 bis einschließlich 6, wobei die Steuerung (112, 1120) dazu konfiguriert ist, basierend auf dem Differenzsignal durch Folgendes zu bestimmen, ob der Aufnahmeverlust der Herzkammer aufgetreten ist:
Bestimmen einer Metrik des Differenzsignals oder einer ersten Ableitung das Differenzsignal, wobei ein größer die Metrik, ein größer die Differenz zwischen der evozierten Reaktionsmorphologie des erfassten EGM und der Polarisationsartefaktmatrize ist und ein kleiner die Metrik, ein geringer die Differenz zwischen der evozierten Reaktionsmorphologie des erfassten EGM und der Polarisationsartefaktmatrize ist;
Vergleichen der Metrik mit einem entsprechenden Schwellenwert; und
Bestimmen, dass der Aufnahmeverlust der Herzkammer aufgetreten ist, wenn die Metrik den entsprechenden Schwellenwert überschreitet.

10. LP (102a, 102b, 1101) nach einem der Ansprüche 1 bis einschließlich 9, wobei der LP (102a, 102b, 1101) einen atrialen LP (102a) umfasst, der dazu konfiguriert ist, in oder auf eine atriale Kammer implantiert zu sein.

11. LP (102a, 102b, 1101) nach einem der Ansprüche 1 bis einschließlich 9, wobei der LP (102a, 102b, 1101) einen ventrikulären LP (102b) umfasst, der dazu konfiguriert ist, in oder auf eine atriale Kammer implantiert zu sein.

12. LP (102a, 102b, 1101) nach einem der Ansprüche 1 bis einschließlich 9, wobei die Steuerung (112, 1120) dazu konfiguriert ist, die Aufnahme-plus-Polarisationsartefaktmatrize durch Mitteln von Abschnitten des erfassten EGM innerhalb jeweiliger Fenster zu produzieren, die folgen, wenn jeder einer Vielzahl der aufnehmenden elektrischen Stimulationsimpulse abgegeben wird.

13. LP (102a, 102b, 1101) nach einem der Ansprüche 1 bis einschließlich 12, wobei die mehreren Elektroden (108a, 108b, 1100, 1102, 1104, 1106, 1108, 1110) lediglich zwei Elektroden (108a, 108b) umfassen, die durch den Impulsgenerator (116, 1122) dazu verwendet werden, die elektrischen Stimulationsimpulse an die Herzkammer abzugeben, und ebenfalls durch die Erfassungsschaltung (132, 1144) dazu verwendet werden, das EGM zu erfassen, das die elektrische Aktivität des Herzens angibt.

14. LP (102a, 102b, 1101) nach Anspruch 13, wobei die lediglich zwei Elektroden (108a, 108b) eine Spitzenelektrode (108a) und eine Ringelektrode (108b) umfassen.

15. LP (102a, 102b, 1101) nach einem der Ansprüche 1 bis einschließlich 12, wobei: die mehreren Elektroden (108a, 108b, 1100, 1102, 1104, 1106, 1108, 1110) drei oder mehr Elektroden umfassen;
ein erster Satz der drei oder mehr Elektroden durch den Impulsgenerator (116, 1122) dazu verwendet wird, die elektrischen Stimulationsimpulse an die Herzkammer abzugeben; und
ein zweiter Satz der drei oder mehr Elektroden, der sich von dem ersten Satz unterscheidet, durch die Erfassungsschaltung (132, 1144) dazu verwendet wird, das EGM zu erfassen, das die elektrische Aktivität des Herzens angibt.

## Revendications

1. Stimulateur cardiaque sans sonde, LP, (102a, 102b, 1101) configuré pour être implanté dans une cavité cardiaque d'un cœur ou sur celle-ci, le LP (102a, 102b, 1101) comprenant :
de multiples électrodes (108a, 108b, 1100, 1102, 1104, 1106, 1108, 1110) ;
une mémoire (160, 1160) ;
un générateur d'impulsions (116, 1122) configuré pour délivrer, en utilisant au moins deux électrodes parmi les multiples électrodes (108a, 108b, 1100, 1102, 1104, 1106, 1108, 1110), des impulsions de stimulation électrique à la cavité cardiaque ;
un circuit de détection (132, 1144) configuré pour détecter, en utilisant au moins deux électrodes parmi les multiples électrodes (108a, 108b, 1100, 1102, 1104, 1106, 1108, 1110), un électrogramme, EGM, indicatif d'une activité électrique du cœur ; et
un dispositif de commande (112, 1120) relié en communication à la mémoire (160, 1160), au générateur d'impulsions (116, 1122) et au circuit de détection (132, 1144), le dispositif de commande (112, 1120) étant configuré pour :
provoquer la délivrance à la cavité cardiaque, en utilisant le générateur d'impulsions (116, 1122) et au moins deux électrodes parmi les multiples électrodes (108a, 108b, 1100, 1102, 1104, 1106, 1108, 1110), d'au moins une impulsion de stimulation électrique de capture ayant un niveau d'énergie d'impulsion de test qui est supérieur à un seuil de capture ;
produire un modèle de capture et d'artéfacts de polarisation sur la base d'au moins une partie de l'EGM détecté dans au moins une fenêtre respective consécutive à l'instant où ladite au moins une impulsion de stimulation électrique de capture a été délivrée ;
sauvegarder, dans une partie de la mémoire (160, 1160), le modèle de capture et d'artéfacts de polarisation correspondant au niveau d'énergie d'impulsion de test ;
provoquer la délivrance à la cavité cardiaque, en utilisant le générateur d'impulsions (116, 1122) et au moins deux électrodes parmi les multiples électrodes (108a, 108b, 1100, 1102, 1104, 1106, 1108, 1110), d'au moins une impulsion de stimulation électrique d'entraînement ayant un niveau d'énergie d'impulsion d'entraînement qui est inférieur au niveau d'énergie d'impulsion de test ;
déterminer un signal de différence entre une morphologie de réponse provoquée de l'EGM détecté et le modèle de capture et d'artéfacts de polarisation, dans lequel la morphologie de réponse provoquée de l'EGM détecté correspond à au moins une partie de l'EGM détecté dans au moins une fenêtre respective consécutive à l'instant où ladite au moins une impulsion de stimulation électrique d'entraînement ayant le niveau d'énergie d'impulsion d'entraînement a été délivrée ; et
déterminer, sur la base du signal de différence, si une perte de capture s'est produite en réponse à ladite au moins une impulsion de stimulation électrique d'entraînement ayant le niveau d'énergie d'entraînement qui est inférieur au niveau d'énergie d'impulsion de test.

2. LP (102a, 102b, 1101) selon la revendication 1, dans lequel le dispositif de commande (112, 1120), en réponse à la détermination du fait qu'une perte de capture de la cavité cardiaque n'est pas survenue en réponse à ladite au moins une impulsion de stimulation électrique d'entraînement ayant le niveau d'énergie d'impulsion d'entraînement qui est inférieur au niveau d'énergie d'impulsion de test, est configuré pour tester un niveau d'énergie d'impulsion d'entraînement diminué en :
provoquant la délivrance à la cavité cardiaque, en utilisant le générateur d'impulsions (116, 1122) et au moins deux électrodes parmi les multiples électrodes (108a, 108b, 1100, 1102, 1104, 1106, 1108, 1110), d'au moins une impulsion de stimulation électrique d'entraînement ayant le niveau d'énergie d'impulsion d'entraînement diminué ;
déterminant un signal de différence supplémentaire entre une morphologie de réponse provoquée supplémentaire de l'EGM détecté et le modèle de capture et d'artéfacts de polarisation, dans lequel la morphologie de réponse provoquée supplémentaire de l'EGM détecté correspond à au moins une partie de l'EGM détecté dans au moins une fenêtre respective consécutive à l'instant où ladite au moins une impulsion de stimulation électrique d'entraînement ayant le niveau d'énergie d'impulsion d'entraînement diminué a été délivrée ; et
déterminant, sur la base du signal de différence supplémentaire, si une perte de capture de la cavité cardiaque s'est produite en réponse à ladite au moins une impulsion de stimulation électrique d'entraînement ayant le niveau d'énergie d'entraînement diminué.

3. LP (102a, 102b, 1101) selon l'une quelconque des revendications 1 et 2, dans lequel le dispositif de commande (112, 1120) est configuré pour :
en réponse à la détermination du fait qu'une perte de capture de la cavité cardiaque n'est pas survenue en réponse à ladite au moins une impulsion de stimulation électrique d'entraînement ayant le niveau d'énergie d'entraînement diminué, tester au moins un niveau d'énergie d'impulsion d'entraînement diminué supplémentaire jusqu'à ce qu'une perte de capture de la cavité cardiaque soit détectée ;
en réponse à la détection d'une perte de capture, identifier en tant que seuil de capture mis à jour un dernier niveau d'énergie d'entraînement diminué ayant provoqué la capture ; et
provoquer la délivrance à la cavité cardiaque, en utilisant le générateur d'impulsions (116, 1122) et au moins deux des multiples électrodes (108a, 108b, 1100, 1102, 1104, 1106, 1108, 1110), d'impulsions de stimulation électrique d'entraînement supplémentaires ayant un niveau d'énergie d'impulsion d'entraînement mis à jour qui dépasse d'une marge de sécurité le seuil de capture mis à jour.

4. LP (102a, 102b, 1101) selon la revendication 3, dans lequel le dispositif de commande (112, 1120) est configuré pour sauvegarder, dans au moins une partie supplémentaire de la mémoire, le seuil de capture mis à jour ou le niveau d'énergie d'impulsion d'entraînement mis à jour, ou les deux.

5. LP (102a, 102b, 1101) selon la revendication 2, dans lequel le dispositif de commande (112, 1120) est configuré pour :
en réponse à la détermination du fait qu'une perte de capture de la cavité cardiaque n'est pas survenue en réponse à ladite au moins une impulsion de stimulation électrique d'entraînement ayant le niveau d'énergie d'entraînement diminué, tester au moins un niveau d'énergie d'impulsion d'entraînement diminué supplémentaire jusqu'à ce qu'une perte de capture de la cavité cardiaque soit détectée ; et
en réponse à la détection d'une perte de capture, provoquer la délivrance à la cavité cardiaque, en utilisant le générateur d'impulsions (116, 1122) et au moins deux des multiples électrodes (108a, 108b, 1100, 1102, 1104, 1106, 1108, 1110), d'impulsions de stimulation électrique d'entraînement supplémentaires ayant un niveau d'énergie d'impulsion d'entraînement mis à jour qui dépasse d'une marge de sécurité un dernier niveau d'énergie d'entraînement diminué ayant provoqué la capture.

6. LP (102a, 102b, 1101) selon l'une quelconque des revendications 1 à 5, dans lequel le dispositif de commande (112, 1120) est configuré pour provoquer la délivrance de ladite au moins une impulsion de stimulation électrique de capture en dehors d'au moins une période réfractaire correspondant à au moins une fenêtre consécutive à au moins une dépolarisation de la cavité cardiaque.

7. LP (102a, 102b, 1101) selon l'une quelconque des revendications 1 à 6, dans lequel le dispositif de commande (112, 1120) est configuré pour déterminer, sur la base du signal de différence, si une perte de capture de la cavité cardiaque s'est produite, en :
déterminant une aire sous la courbe du signal de différence ou d'une première dérivée du signal de différence ;
comparant l'aire sous la courbe à un seuil correspondant ; et
déterminant qu'une perte de capture de la cavité cardiaque s'est produite lorsque l'aire sous la courbe dépasse le seuil correspondant.

8. LP (102a, 102b, 1101) selon l'une quelconque des revendications 1 à 6, dans lequel le dispositif de commande (112, 1120) est configuré pour déterminer, sur la base du signal de différence, si une perte de capture de la cavité cardiaque s'est produite, en :
déterminant une première dérivée du signal de différence ;
identifiant un pic maximal de la première dérivée du signal de différence ;
identifiant une fenêtre de la première dérivée du signal de différence qui est centrée sur le pic maximal ;
déterminant une aire sous la courbe pour la fenêtre de la première dérivée du signal de différence qui est centrée sur le pic maximal ;
comparant l'aire sous la courbe à un seuil correspondant ; et
déterminant qu'une perte de capture de la cavité cardiaque s'est produite lorsque l'aire sous la courbe dépasse le seuil correspondant.

9. LP (102a, 102b, 1101) selon l'une quelconque des revendications 1 à 6, dans lequel le dispositif de commande (112, 1120) est configuré pour déterminer, sur la base du signal de différence, si une perte de capture de la cavité cardiaque s'est produite, en :
déterminant une métrique du signal de différence ou d'une première dérivée du signal de différence, dans lequel plus la métrique est grande, plus la différence entre la morphologie de réponse provoquée de l'EGM détecté et le modèle d'artéfacts de polarisation est grande, et plus la métrique est petite, plus la différence entre la morphologie de réponse provoquée de l'EGM détecté et le modèle d'artéfacts de polarisation est petite ;
comparant la métrique à un seuil correspondant ; et
déterminant qu'une perte de capture de la cavité cardiaque s'est produite lorsque la métrique dépasse le seuil correspondant.

10. LP (102a, 102b, 1101) selon l'une quelconque des revendications 1 à 9, dans lequel le LP (102a, 102b, 1101) comprend un LP auriculaire (102a) configuré pour être implanté dans une cavité auriculaire ou sur celle-ci.

11. LP (102a, 102b, 1101) selon l'une quelconque des revendications 1 à 9, dans lequel le LP (102a, 102b, 1101) comprend un LP ventriculaire (102b) configuré pour être implanté dans une cavité auriculaire ou sur celle-ci.

12. LP (102a, 102b, 1101) selon l'une quelconque des revendications 1 à 9, dans lequel le dispositif de commande (112, 1120) est configuré pour produire le modèle de capture et d'artéfacts de polarisation en calculant la moyenne de parties de l'EGM détecté dans des fenêtres respectives consécutives à l'instant où chacune d'une pluralité des impulsions de stimulation électrique de capture est délivrée.

13. LP (102a, 102b, 1101) selon l'une quelconque des revendications 1 à 12, dans lequel les multiples électrodes (108a, 108b, 1100, 1102, 1104, 1106, 1108, 1110) comprennent seulement deux électrodes (108a, 108b) qui sont utilisées par le générateur d'impulsions (116, 1122) pour délivrer les impulsions de stimulation électrique à la cavité cardiaque et qui sont également utilisées par le circuit de détection (132, 1144) pour détecter l'EGM indicatif de l'activité électrique du cœur.

14. LP (102a, 102b, 1101) selon la revendication 13, dans lequel les seules deux électrodes (108a, 108b) comprennent une électrode de pointe (108a) et une électrode annulaire (108b).

15. LP (102a, 102b, 1101) selon l'une quelconque des revendications 1 à 12, dans lequel : les multiples électrodes (108a, 108b, 1100, 1102, 1104, 1106, 1108, 1110) comprennent au moins trois électrodes ;
un premier ensemble desdites au moins trois électrodes est utilisé par le générateur d'impulsions (116, 1122) pour délivrer les impulsions de stimulation électrique à la cavité cardiaque ; et
un second ensemble desdites au moins trois électrodes qui diffère du premier ensemble est utilisé par le circuit de détection (132, 1144) pour détecter l'EGM indicatif de l'activité électrique du cœur.
